# EUROPEAN PATENT APPLICATION

(11) **EP 4 413 867 A1**
(43) Date of publication of application: **14.08.2024**
(21) Application number: 22837769.3
(22) Date of filing: 08.07.2022
(51) Int. Cl.: A23K 20/147, A01N 63/50, A01P 3/00, A23K 10/16, A23K 20/137, A23K 20/195, A61K 35/747, A61K 38/16, A61P 31/04, C07K 14/195, C07K 14/335, C12N 1/20

(54) **FISH REARING COMPOSITION, AND COMPOSITION FOR TREATING OR PREVENTING FISH DISEASES**

(30) Priority: 09.07.2021 JP 2021114166
(71) Applicant: Sumitomo Chemical Company, Limited, Tokyo 103-6020 (JP); Tokyo University of Marine Science and Technology, Tokyo 108-8477 (JP)
(72) Inventor: AOKI, Mikio, Osaka-shi, Osaka 554-8558 (JP); MIKATA, Kazuki, Osaka-shi, Osaka 554-8558 (JP); KAI, Toshihiro, Osaka-shi, Osaka 554-8558 (JP); KUWAHARA, Hiroshi, Osaka-shi, Osaka 554-8558 (JP); HIRONO, Ikuo, Tokyo 108-8477 (JP); KONDO, Hidehiro, Tokyo 108-8477 (JP)
(74) Representative: Vossius & Partner Patentanwälte Rechtsanwälte mbB
(86) International application number: PCT/JP2022/027146
(87) International publication number: WO 2023/282355

(57) **Abstract**

Provided are: a fish rearing composition containing at least one selected from plantaricin and nisin; and a fish rearing composition containing bacterial cells or a cell culture product of a bacterium having at least one selected from a plantaricin gene and a nisin gene, or an extract thereof. Provided is a method for rearing an organism of fish, including administering bacterial cells or a cell culture product of a bacterium having at least one selected from a plantaricin gene and a nisin gene, or an extract thereof.

## Description

### TECHNICAL FILED

The present invention relates to a fish rearing composition, and a composition for preventing or treating a fish disease. The present invention also relates to a method for rearing an organism of fish with the composition.

### BACKGROUND ART

*Lactococcus garvieae* infects organisms of fish and causes streptococcosis. Streptococcosis is a fish disease causing the largest amount of damage in the aquaculture of Japanese yellowtail. Conventionally, vaccine injection to juvenile fish has been performed in advance for the epidemic of streptococcosis. However, newtype pathogenic bacteria have appeared and are causing a problem of failure in preventing the onset of streptococcosis even with vaccine administration.

Daniel Vendrell et al, Comp Immunol Microbiol Infect Dis. 2008 Jul; 31(4):337-45 (NPL 1) and Tania Perez-Sanchez et al, Fish & Shellfish Immunology 31 (2011) 196-201 (NPL 2) disclose that living bacterial cells of the *Lactobacillus plantarum* strains CLFP3 and CLFP238 each activated the immune response of rainbow trout and reduced the number of rainbow trout killed by *Lactococcus garvieae.* Cynthia Sequeiros et al, Arch Microbiol (2015) 197:449-458 (NPL 3) discloses that a strain of the lactic acid bacterium *Lactococcus lactis,* TW34, produces nisin Z, and nisin Z inhibited the growth of the *Lactococcus garvieae* 03/8460 strain. Carlos Araujo et al, Mar Biotechnol (2015) 17:820-830 (NPL 4) discloses that a strain of the lactic acid bacterium *Lactococcus lactis* subsp. *cremoris,* WA2-67, produces nisin Z, nisin Z inhibited the growth of the *Lactococcus garvieae* CLG4 strain, and living bacterial cells of the WA2-67 strain reduced the number of rainbow trout killed by *Lactococcus garvieae.*

### CITATION LIST

### NON PATENT LITERATURE

NPL 1: Daniel Vendrell et al, Comp Immunol Microbiol Infect Dis. 2008 Jul; 31(4):337-45
NPL 2: Tania Perez-Sanchez et al, Fish & Shellfish Immunology 31 (2011) 196-201
NPL 3: Cynthia Sequeiros et al, Arch Microbiol (2015) 197:449-458
NPL 4: Carlos Araujo et al, Mar Biotechnol (2015) 17:820-830

### SUMMARY OF INVENTION

### TECHNICAL PROBLEM

An object of the present invention is to provide a novel composition and rearing method to inhibit fish diseases.

### SOLUTION TO PROBLEM

The present invention relates to items exemplified in the following.
[1] A composition for rearing an organism of fish, comprising at least one selected from plantaricin and nisin.
[2] A composition for preventing or treating a disease caused by *Lactococcus garvieae* in fish, comprising at least one selected from plantaricin and nisin.
[3] The composition according to [1] or [2], wherein
   the plantaricin is at least one selected from plantaricin A, plantaricin E, plantaricin F, plantaricin J, plantaricin K, plantaricin NC8α, and plantaricin NC8β, and
   the nisin is at least one selected from nisin A and nisin Z.
[4] The composition according to any of [1] to [3], wherein the plantaricin contains any sequence of (a) to (c) and contains a peptide having antibacterial activity:
   (a) an amino acid sequence set forth in any of SEQ ID NOs: 1 to 7,
   (b) an amino acid sequence having 85% or more identity with an amino acid sequence set forth in any of SEQ ID NOs: 1 to 7, and
   (c) an amino acid sequence formed by deletion, substitution, insertion, and/or addition of 1 or more and 15 or less amino acid residues in an amino acid sequence set forth in any of SEQ ID NOs: 1 to 7.
[5] A composition for rearing an organism of fish, comprising bacterial cells or a cell culture product of a bacterium having at least one selected from a plantaricin gene and a nisin gene, or an extract thereof.
[6] A composition for preventing or treating a disease caused by *Lactococcus garvieae* in fish, comprising bacterial cells or a cell culture product of a bacterium having at least one selected from a plantaricin gene and a nisin gene, or an extract thereof.
[7] The composition according to [5] or [6], wherein the bacterium having at least one selected from the plantaricin gene and the nisin gene is at least one selected from NITE-BP-03198, NITE-BP-03199, NITE-BP-03200, NITE-BP-03201, NITE-BP-03202, and NITE-ABP-03481.
[8] The composition according to [5] or [6], wherein
   the plantaricin gene is at least one selected from a plantaricin A gene, a plantaricin E gene, a plantaricin F gene, a plantaricin J gene, a plantaricin K gene, a plantaricin NC8α gene, and a plantaricin NC8β gene, and
   the nisin gene is at least one selected from a nisin A gene and a nisin Z gene.
[9] The composition according to any of [1] to [8], wherein the fish belongs to the order Perciformes or the order Salmoniformes.
[10] The composition according to any of [1] to [9], wherein the composition is a feed or a feed additive.
[11] The composition according to any of [1] to [9], wherein the composition is rearing water or a rearing water additive.
[12] The composition according to any of [1] to [9], wherein the composition is an injection.
[13] A method for rearing an organism of fish, comprising giving the composition according to any of [1] to [11] to an organism of fish.
[14] A method for rearing an organism of fish, comprising administering at least one selected from plantaricin and nisin to an organism of fish.
[15] A method for preventing or treating a disease caused by *Lactococcus garvieae* in fish, comprising administering at least one selected from plantaricin and nisin to an organism of fish.
[16] The method according to [14] or [15], wherein the plantaricin is at least one selected from plantaricin A, plantaricin E, plantaricin F, plantaricin J, plantaricin K, plantaricin NC8α, and plantaricin NC8β, and
   the nisin is at least one selected from nisin A and nisin Z.
[17] The method according to any of [14] to [16], wherein the plantaricin contains any sequence of (a) to (c) and contains a peptide having antibacterial activity:
   (a) an amino acid sequence set forth in any of SEQ ID NOs: 1 to 7,
   (b) an amino acid sequence having 85% or more identity with an amino acid sequence set forth in any of SEQ ID NOs: 1 to 7, and
   (c) an amino acid sequence formed by deletion, substitution, insertion, and/or addition of 1 or more and 15 or less amino acid residues in an amino acid sequence set forth in any of SEQ ID NOs: 1 to 7.
[18] A method for rearing an organism of fish, comprising administering bacterial cells or a cell culture product of a bacterium having at least one selected from a plantaricin gene and a nisin gene, or an extract thereof to an organism of fish.
[19] A method for preventing or treating a disease caused by *Lactococcus garvieae* in fish, comprising administering bacterial cells or a cell culture product of a bacterium having at least one selected from a plantaricin gene and a nisin gene, or an extract thereof to an organism of fish.
[20] The method according to [18] or [19], wherein the bacterium having at least one selected from the plantaricin gene and the nisin gene is at least one selected from NITE-BP-03198, NITE-BP-03199, NITE-BP-03200, NITE-BP-03201, NITE-BP-03202, and NITE-ABP-03481.
[21] The method according to [18] or [19], wherein
   the plantaricin gene is at least one selected from a plantaricin A gene, a plantaricin E gene, a plantaricin F gene, a plantaricin J gene, a plantaricin K gene, a plantaricin NC8α gene, and a plantaricin NC8β gene, and
   the nisin gene is at least one selected from a nisin A gene and a nisin Z gene.
[22] The method according to any of [14] to [21], wherein the administering is oral administration, soaking administration, or injection administration.
[23] The method according to any of [14] to [22], wherein the fish belongs to the order Perciformes or the order Salmoniformes.
[24] A growth inhibitor or a bactericide for *Lactococcus garvieae,* comprising at least one selected from plantaricin and nisin.
[25] A growth-inhibiting or bactericidal method for *Lactococcus garvieae,* comprising bringing at least one selected from plantaricin and nisin into contact with *Lactococcus garvieae.*
[26] A growth inhibitor or a bactericide for *Lactococcus garvieae,* comprising bacterial cells or a cell culture product of a bacterium having at least one selected from a plantaricin gene and a nisin gene, or an extract thereof.
[27] A growth-inhibiting or bactericidal method for *Lactococcus garvieae,* comprising bringing bacterial cells or a cell culture product of a bacterium having at least one selected from a plantaricin gene and a nisin gene, or an extract thereof into contact with *Lactococcus garvieae.*
[28] A bacterium of Receipt Number: NITE-ABP-03481.
[29] Bacterial cells or a cell culture product of the bacterium according to [28], or an extract thereof.

### ADVANTAGEOUS EFFECTS OF INVENTION

The present invention can provide a novel composition and rearing method to inhibit fish diseases.

### BRIEF DESCRIPTION OF DRAWINGS

Fig. 1 shows photographs showing (A) the colony shape of and (B) a result of Gram staining of NITE BP-03198 in Experiment 1.
Fig. 2 shows photographs showing (A) the colony shape of and (B) a result of Gram staining of NITE BP-03199 in Experiment 2.
Fig. 3 shows photographs showing (A) the colony shape of and (B) a result of Gram staining of NITE BP-03200 in Experiment 3.
Fig. 4 shows photographs showing (A) the colony shape of and (B) a result of Gram staining of NITE BP-03201 in Experiment 4.
Fig. 5 shows photographs showing (A) the colony shape of and (B) a result of Gram staining of NITE BP-03202 in Experiment 5.
Fig. 6 shows photographs showing (A) the colony shape of and (B) a result of Gram staining of NITE ABP-03481 in Experiment 6.
Fig. 7 shows a diagram illustrating test for evaluating antibacterial activity *to Lactococcus garvieae* in Experiment 7.

### DESCRIPTION OF EMBODIMENTS

Hereinafter, modes of implementation of the present invention will be described in detail. The present invention is not limited to the following embodiments.

Herein, each expression in the format "A to B" indicates the upper limit and lower limit of a range (i.e., A or more and B or less), and if a unit is shown only for B but not for A, the units of A and B are the same.

### [Compositions for rearing organism of fish]

A composition for rearing an organism of fish according to an embodiment of the present invention contains at least one selected from plantaricin and nisin. A composition for rearing an organism of fish according to an embodiment of the present invention contains bacterial cells or a cell culture product of a bacterium having at least one selected from a plantaricin gene and a nisin gene, or an extract thereof. The compositions for rearing an organism of fish according the present invention can inhibit streptococcosis caused by *Lactococcus garvieae* in fish.

The plantaricin may be at least one selected from plantaricin A, plantaricin E, plantaricin F, plantaricin J, plantaricin K, plantaricin NC8α, and plantaricin NC8β. The nisin may be at least one selected from nisin A and nisin Z.

The plantaricin may be a peptide containing any sequence of (a) to (c) below. The plantaricin has antibacterial activity to *Lactococcus garvieae.*
(a) An amino acid sequence set forth in any of SEQ ID NOs: 1 to 7,
(b) an amino acid sequence having 85% or more identity with an amino acid sequence set forth in any of SEQ ID NOs: 1 to 7, and
(c) an amino acid sequence formed by deletion, substitution, insertion, and/or addition of 1 or more and 15 or less amino acid residues in an amino acid sequence set forth in any of SEQ ID NOs: 1 to 7.

The plantaricin may be a peptide consisting of an amino acid sequence set forth in any of SEQ ID NOs: 1 to 7.

SEQ ID NOs: 1 to 7 show part or the whole of mature peptides of plantaricin A, plantaricin E, plantaricin F, plantaricin J, plantaricin K, plantaricin NC8α, and plantaricin NC8β, which are predicted from the genome sequences of NITE-BP-03198, NITE-BP-03199, NITE-BP-03200, NITE-BP-03201, and NITE-BP-03202, respectively.

The plantaricin may be a peptide containing an amino acid sequence having, for example, 90% or more, 95% or more, 96% or more, 97% or more, 98% or more, or 99% or more identity with an amino acid sequence set forth in any of SEQ ID NOs: 1 to 7. The upper limit of the identity of the amino acid sequence is not limited, and the identity may be, for example, 100% or less.

The plantaricin may be a peptide containing an amino acid sequence formed by deletion, substitution, insertion, and/or addition of, for example, 1 or more and 12 or less, 10 or less, 9 or less, 8 or less, 7 or less, 6 or less, 5 or less, 4 or less, 3 or less, or 2 or less amino acid residues in an amino acid sequence set forth in any of SEQ ID NOs: 1 to 7.

An example of substitution, deletion, insertion, and/or addition of an amino acid residue is conservative mutation, through which normal protein functions are maintained. A representative example of conservative mutation is conservative substitution. Conservative substitution is mutation to cause mutual substitution among Phe, Trp, and Tyr for aromatic amino acids at the site of substitution, among Leu, Ile, and Val for hydrophobic amino acids at the site of substitution, between Gin and Asn for polar amino acids, among Lys, Arg, and His for basic amino acids, between Asp and Glu for acidic amino acids, and between Ser and Thr for amino acids having a hydroxyl group. Examples of substitution regarded as conservative substitution include substitution of Ala with Ser or Thr, substitution of Arg with Gln, His, or Lys, substitution of Asn with Glu, Gln, Lys, His, or Asp, substitution of Asp with Asn, Glu, or Gln, substitution of Cys with Ser or Ala, substitution of Gin with Asn, Glu, Lys, His, Asp, or Arg, substitution of Glu with Gly, Asn, Gln, Lys, or Asp, substitution of Gly with Pro, substitution of His with Asn, Lys, Gln, Arg, or Tyr, substitution of Ile with Leu, Met, Val, or Phe, substitution of Leu with Ile, Met, Val, or Phe, substitution of Lys with Asn, Glu, Gln, His, or Arg, substitution of Met with Ile, Leu, Val, or Phe, substitution of Phe with Trp, Tyr, Met, Ile, or Leu, substitution of Ser with Thr or Ala, substitution of Thr with Ser or Ala, substitution of Trp with Phe or Tyr, substitution of Tyr with His, Phe, or Trp, and substitution of Val with Met, Ile, or Leu.

In a mode, the plantaricin may be a peptide having an amino acid sequence formed by adding part or the whole of Trp-Gly-Trp to the C terminus of the amino acid sequence set forth in SEQ ID NO: 1.

In a mode, the plantaricin may be a peptide having an amino acid sequence formed by adding part or the whole of Phe-Asn-Arg-Gly-Gly-Tyr-Asn, part or the whole of Gly-Gly-Phe-Asn-Arg-Gly-Gly-Tyr-Asn, part or the whole of Phe-Asn-Trp-Gly-Gly-Tyr-Asn, part or the whole of Phe-Asn-Arg-Asp-Gly-Tyr-Asn, or part or the whole of Phe-Asn-Arg-Val-Gly-Tyr-Asn to the N terminus of the amino acid sequence set forth in SEQ ID NO: 2. The plantaricin may be a peptide having an amino acid sequence formed by adding part or the whole of Lys-Ser-Ile-Arg or part or the whole of Asn-Ile-Arg to the C terminus of the amino acid sequence set forth in SEQ ID NO: 2.

In a mode, the plantaricin may be a peptide having an amino acid sequence formed by adding part or the whole of Val-Ile-Ser-Ala-Val-Arg-Gly-Phe-Ile-His-Gly, part or the whole of Ile-Ile-Ser-Ala-Val-Arg-Gly-Phe-Ile-His-Gly, or part or the whole of Val-Ile-Ser-Ala-Ile-Arg-Gly-Phe-Ile-His-Gly to the C terminus of the amino acid sequence set forth in SEQ ID NO: 3.

In a mode, the plantaricin may be a peptide having an amino acid sequence formed by adding part or the whole of Ile-Arg-Arg, part or the whole of Leu-Arg-Arg, or part or the whole of Asn-Pro-Ser-Leu-Ile-Asn-Gly-Leu-Lys-Leu-Arg-Arg to the C terminus of the amino acid sequence set forth in SEQ ID NO: 4. The plantaricin may be a peptide having an amino acid sequence formed by substituting Gly at position 1 with Ser or Asp, Lys at position 4 with Glu, Gly at position 13 with Glu, and Ala at position 19 with Thr in the amino acid sequence set forth in SEQ ID NO: 4.

In a mode, the plantaricin may be a peptide having an amino acid sequence formed by substituting Arg at position 2 with Trp in the amino acid sequence set forth in SEQ ID NO: 5.

In a mode, the plantaricin may be a peptide having an amino acid sequence formed by substituting Val at position 27 with Ala in the amino acid sequence set forth in SEQ ID NO: 6. The plantaricin may be a peptide having an amino acid sequence formed by adding Phe to the C terminus of the amino acid sequence set forth in SEQ ID NO: 6.

The plantaricin and the nisin may be each synthesized one, or one produced in a microorganism having a plantaricin gene or a nisin gene. In an aspect of the present embodiment, the plantaricin and the nisin may be ones synthesized by using a technique of organic synthesis, or ones synthesized by using a gene engineering technique. The plantaricin gene and the nisin gene may be endogenous or exogenous.

The plantaricin gene may be at least one selected from a plantaricin A gene (e.g., a gene having a nucleotide sequence set forth in SEQ ID NO: 20, 30, or 40), a plantaricin E gene (e.g., a gene having a nucleotide sequence set forth in SEQ ID NO: 8, 22, 32, 42, or 50), a plantaricin F gene (e.g., a gene having a nucleotide sequence set forth in SEQ ID NO: 10, 24, 34, 44, or 52), a plantaricin J gene (e.g., a gene having a nucleotide sequence set forth in SEQ ID NO: 12, 26, 46, or 54), a plantaricin K gene (e.g., a gene having a nucleotide sequence set forth in SEQ ID NO: 14, 28, 48, or 56), a plantaricin NC8α gene (e.g., a gene having a nucleotide sequence set forth in SEQ ID NO: 16, 36, or 58), and a plantaricin NC8β gene (e.g., a gene having a nucleotide sequence set forth in SEQ ID NO: 18, 38, or 60). The nisin gene may be at least one selected from a nisin A gene and a nisin Z gene (e.g., a gene having a nucleotide sequence set forth in SEQ ID NO: 62).

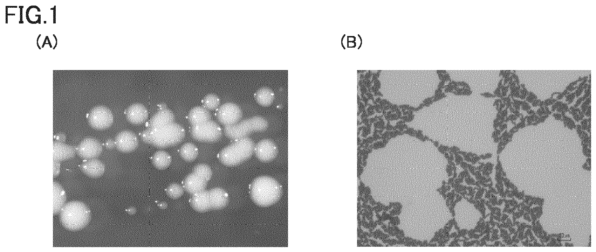

Examples of bacteria having a plantaricin gene include NITE-BP-03198, NITE-BP-03199, NITE-BP-03200, NITE-BP-03201, and NITE-BP-03202. NITE-BP-03198, NITE-BP-03199, NITE-BP-03200, NITE-BP-03201, and NITE-BP-03202 are bacteria internationally deposited in the National Institute of Technology and Evaluation NITE Patent Microorganisms Depositary (NPMD, Address: Room 122, 2-5-8, Kazusakamatari, Kisarazu, Chiba, Japan 292-0818) in accordance with the Budapest Treaty under Accession Number: NITE BP-03198 (Original date of deposit: April 9, 2020), Accession Number: NITE BP-03199 (Original date of deposit: April 9, 2020), Accession Number: NITE BP-03200 (Original date of deposit: April 9, 2020), Accession Number: NITE BP-03201 (Original date of deposit: April 9, 2020), and Accession Number: NITE BP-03202 (Original date of deposit: April 9, 2020), respectively. The bacteria are each a bacterium belonging to *Lactobacillus plantarum,* which is one of lactic acid bacteria. The bacteriological characteristics of the bacteria are shown in Table 1 to Table 10 and Fig. 1 to Fig. 5 presented later.

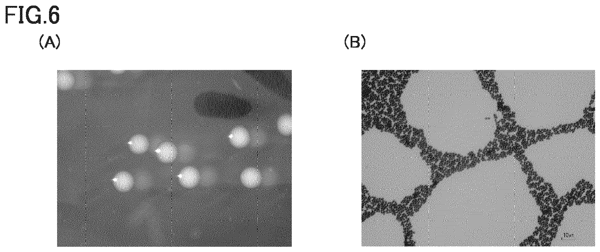

Examples of bacteria having a nisin gene include ATCC-11454 and NITE-ABP-03481. ATCC-11454 is a bacterium belonging to *Lactococcus lactis* subsp. *lactis,* which is one of lactic acid bacteria. NITE-ABP-03481 is a bacterium internationally deposited in the National Institute of Technology and Evaluation NITE Patent Microorganisms Depositary (NPMD, Address: Room 122, 2-5-8, Kazusakamatari, Kisarazu, Chiba, Japan 292-0818) in accordance with the Budapest Treaty under Receipt Number: NITE ABP-03481 (Date of receipt: June 9, 2021). NITE-ABP-03481 is a bacterium belonging to *Lactococcus lactis,* which is one of lactic acid bacteria. The bacteriological characteristics of NITE ABP-03481 are shown in Table 11 and Table 12 and Fig. 6 presented later.

NITE-BP-03198 is present in fermented foods and NITE-BP-03199, NITE-BP-03200, NITE-BP-03201, NITE-BP-03202, and NITE-ABP-03481 are present in the natural environment, and hence they are inferred to cause less impact on the environment in using for rearing an organism of fish and to be safe for humans. Each of the bacteria may be an isolated bacterium.

A composition for rearing an organism of fish according to an embodiment of the present invention contains bacterial cells or a cell culture product of at least one bacterium selected from NITE-BP-03198, NITE-BP-03199, NITE-BP-03200, NITE-BP-03201, NITE-BP-03202, and NITE-ABP-03481, or an extract thereof. The bacterial cells may be cells isolated from a fermented food or from the environment, or cultured one. The bacterial cells may be dead bacterial cells or living bacterial cells. The bacterial cells may be cells in a culture solution, a buffer solution, or the like, or in a product obtained by concentrating the culture solution, buffer solution, or the like to remove the liquid or a freeze-dried product thereof, or in a frozen stock.

The cell culture product may contain secretions, metabolites, and others from the bacterium. In the cell culture product, peptides, proteins, saccharides, enzymes, and organic acids produced by the bacterium, and medium (liquid medium or solid medium) containing any of them can be contained. The cell culture product may be the supernatant after culture of the bacterium. The culture supernatant can be obtained, for example, by removing the bacterium from the liquid medium in which the bacterium was cultured through centrifugation, filtration operations, or the like.

NITE-BP-03198, NITE-BP-03199, NITE-BP-03200, NITE-BP-03201, NITE-BP-03202, and NITE-ABP-03481 can be cultured according to a common culture method for lactic acid bacteria. A representative culture method is, for example, a method of culturing with MRS (de Man, Rogosa and Sharpe) liquid medium or MRS agar medium at a temperature of 30°C.

The extract of the bacterial cells or the cell culture product is prepared in such a manner that the antibacterial activity of the bacterial cells or the cell culture product of the lactic acid bacterium to *Lactococcus garvieae* is not lost. The extract can be obtained, for example, by treating the bacterial cells or the cell culture product of the bacterium through ultrasonic homogenization, bead grinding, freeze-thawing, or chemical dissolution. The extract may be obtained, for example, by performing salting-out, ultrafiltration, ion-exchange chromatography, or liquid-phase extraction with organic solvent on the bacterial cells or the cell culture product. These operations can be performed in combination, as appropriate. The extract can contain bacterial cell fragments, nucleic acids, peptides, proteins, saccharides, and enzymes from the bacterium. Herein, the bacterial cells or the cell culture product of the bacterium, or the extract thereof is also referred to as the "bacterial cell preparation".

Herein, examples of the fish include, but are not limited to, the order Perciformes, the order Salmoniformes, the order Tetraodontiformes, the order Pleuronectiformes, the order Anguilliformes, the order Clupeiformes, the order Gadiformes, the order Scorpaeniformes, the order Cypriniformes, the order Clupeiformes, and the order Mugiliformes. The order Perciformes includes the family Carangidae, the family Scombridae, the family Lateolabracidae, the family Sparidae, the family Sebastidae, the family Triglidae, the family Hexagrammidae, the family Cottidae, the family Istiophoridae, the family Sphyraenidae, the family Trichiuridae, the family Latidae, the family Haemulidae, the family Oplegnathidae, the family Sillaginidae, the family Priacanthidae, the family Epinephelidae, the family Mullidae, the family Scaridae, and the family Labridae. The family Carangidae includes the genus *Seriola* (e.g., Japanese yellowtail, Great amberjack, Yellowtail amberjack, Almaco jack), the genus *Pseudocaranx* (e.g., striped jack), and the genus *Trachurus* (e.g., Japanese jack mackerel). The family Scombridae incudes the genus *Scomber* (e.g., chub mackerel, blue mackerel, Atlantic mackerel) and the genus *Thunnus* (e.g., Pacific bluefin tuna, Northern bluefin tuna, yellowfin tuna). The family Lateolabracidae includes the genus *Lateolabrax* (e.g., Japanese sea bass). The family Sparidae includes the genus *Rhabdosargus* (e.g., Goldlined seabream), the genus *Pagrus* (e.g., red seabream), and the genus *Evynnis* (e.g., crimson sea bream). The order Salmoniformes includes the family Salmonidae. The family Salmonidae includes the genus *Salmo* (e.g., rainbow trout, Atlantic salmon) and the genus *Oncorhynchus* (e.g., chum salmon, coho salmon). The order Tetraodontiformes includes the family Tetraodontidae and the family Monacanthidae. The family Monacanthidae includes the genus *Stephanolepis* (e.g., filefish) and the genus *Thamnaconus* (e.g., black scraper).

Each of the compositions for rearing an organism of fish may be a composition for oral administration. The composition for oral administration may be any composition to be orally administered to an organism of fish, without limitation. Each of the fish rearing composition may be a feed for fish (hereinafter, also referred to as a "fish feed") or a feed additive. The composition for oral administration may be administered to an environment in which an organism of fish is reared (e.g., rearing water) and orally ingested by the organism of fish reared in the environment, into which plantaricin, nisin, the lactic acid bacterial cell preparation, and others are released by dissolution. The composition for oral administration to fish can inhibit infections caused by *Lactococcus garvieae* in an organism of fish to which the composition has been administered. The composition for oral administration according to the present invention gives reduced burdens in administration as compared with conventional methods for inhibiting fish diseases, which involve fish-by-fish vaccine injection. In addition, the composition for oral administration according to the present invention is not limited to specific fish species, and applicable to rearing of a wide variety of biological species.

The fish feed may contain any component that is typically used for rearing or aquaculture of organisms of fish, and may be produced by any production method. An appropriate composition can be select for the fish feed, depending on the type and growth stage of the fish. The fish feed may contain a protein source such as squid meal, krill meal, fish meal, soybean meal, and corn gluten meal, and a binder such as gluten and starch. The fish feed may contain a known carrier or additive acceptable for feeds, and may contain, for example, a medicament for aquatic organisms such as an erythromycin preparation, an ampicillin preparation, a praziquantel preparation, a lysozyme chloride preparation, an oxytetracycline hydrochloride preparation, a spiramycin preparation, a sodium nifurstyrenate preparation, a lincomycin hydrochloride preparation, a flumequine preparation, and a glutathione preparation, a nutritional supplement substance such as a vitamin such as vitamin C, vitamin B1, vitamin A, vitamin D, and vitamin E, and an amino acid such as lysine, methionine, and histidine, a pigment such as β-carotene, astaxanthin, and canthaxanthin, a mineral such as calcium and silicate, a trace metal, and a preservative.

The fish feed may have any shape and size, depending on the type and size or the like of the fish to be reared. For example, the fish feed may be a powdery feed obtained by mixing and pulverizing dry raw materials, a solidified feed (dry pellets) obtained by solidifying a powder, a pasty feed containing moisture (moist pellets), or a raw feed (slices of fish). In an example of methods for producing the fish feed, first, a common powdery feed for fish aquaculture, and plantaricin, nisin, or the lactic acid bacterial cell preparation are mixed together. This mixture is molded, for example, extrusion-molded by using a pasta machine or a syringe. Finally, the molded product is dried, for example, at 60°C to 65°C for about 2 hours, giving a fish feed containing plantaricin, nisin, or the lactic acid bacterial cell preparation. Other examples of the fish feed include a product obtained by dredging plantaricin, nisin, or the lactic acid bacterial cell preparation over a common powdery feed for fish aquaculture and a product obtained by soaking a powdery feed for fish aquaculture in a liquid containing plantaricin, nisin, or the lactic acid bacterial cell preparation (e.g., culture supernatant).

The amount of plantaricin, nisin, or the lactic acid bacterial cell preparation contained in the fish feed is not limited as long as the antibacterial activity to *Lactococcus garvieae* is allowed to be exhibited, and may be an amount suitable for the bacterial cell density of *Lactococcus garvieae,* the rearing place, the rearing temperature, the oxygen concentration, the rearing density, the type of the fish of interest, and so on. The total amount of the lactic acid bacterial cell preparation in the fish feed may be, for example, 1 × 10³ to 1 × 10¹² cfu (colony-forming unit)/g, or 1 × 10⁴ to 1 × 10¹² cfu/g, or 1 × 10⁵ to 1 × 10¹² cfu/g. The colony-forming unit can be determined by an agar plate culture method. The total amount of plantaricin, nisin, and the lactic acid bacterial cell preparation (content ratio) may be 0.001% by mass to 50% by mass, preferably 0.001% by mass to 30% by mass, more preferably 0.01% by mass to 30% by mass, and even more preferably 0.01% by mass to 10% by mass to the feed weight. The total amount can be measured by weighing with a balance or the like.

The fish feed additive may be any additive that can be added to common fish feeds for aquaculture, without limitation. The feed additive may be liquid or powder, and may be freeze-dried. The feed additive may be plantaricin, nisin, or the lactic acid bacterial cell preparation as it is. The feed additive may contain a liquid, a spreader, or the like for making it easy to allow plantaricin, nisin, or the lactic acid bacterial cell preparation to be adhered to, absorbed in, or mixed with a fish feed. It is preferable that the feed additive be added to a feed in such a manner that the total amount of plantaricin, nisin, and the bacterial cell preparation contained in the feed falls within the range shown above.

The feed containing plantaricin, nisin, or the lactic acid bacterial cell preparation may be fed once or in multiple portions per day. The feed containing plantaricin, nisin, or the lactic acid bacterial cell preparation may be fed once every several days. An appropriate period can be selected for feeding the feed containing plantaricin, nisin, or the lactic acid bacterial cell preparation, for example, depending on the type of the fish of interest. The feed containing plantaricin, nisin, or the lactic acid bacterial cell preparation may be continuously fed for the whole period of aquaculture (rearing), or fed only for part of the period. The "part of the period" may be, for example, 10% or more, 20% or more, 30% or more, 50% or more, 70% or more, or 90% or more of the whole period of rearing. In an aspect of the present embodiment, the upper limit value of the "part of the period" is not limited, and the part of the period may be, for example, less than 100% of the whole period of rearing, or 99% or less thereof. The period to feed the feed containing plantaricin, nisin, or the lactic acid bacterial cell preparation may be, for example, 1 month to 3 years. The feed containing plantaricin, nisin, or the lactic acid bacterial cell preparation may be fed in cycles of continuing feeding and suspending each for any period.

The compositions for rearing an organism of fish may be given to an organism of fish. Examples of methods for giving any of the compositions for rearing an organism of fish to an organism of fish include methods of mixing water to rear an organism of fish and the composition for rearing an organism of fish. Examples of the methods of mixing water to rear an organism of fish and the composition for rearing an organism of fish include a method of dropping the composition for rearing an organism of fish into water, a method of pouring water into a rearing tank in which the composition for rearing an organism of fish is placed, and a method of adding the composition for rearing an organism of fish, dissolved in water, to water to rear an organism of fish. The composition for rearing an organism of fish may be automatically given to an organism of fish at constant intervals.

Each of the compositions for rearing an organism of fish may be a composition for soaking administration. The composition for soaking administration may be rearing water or a rearing water additive. The rearing water may be a liquid that is commonly used for rearing organisms of fish, and may be freshwater, brackish water, or seawater (including artificially prepared seawater). Rearing of an organism of fish may be performed in a natural environment, in an aquaculture pond, or in an aquarium. The rearing water may be aerated. The rearing water, which contains plantaricin, nisin, or the lactic acid bacterial cell preparation, has antibacterial activity to *Lactococcus garvieae.* Organisms of fish soaked in the rearing water can be prevented from being affected by infections caused by *Lactococcus garvieae.* The composition for soaking administration can inhibit infections caused by *Lactococcus garvieae* in an organism of fish to which the composition has been administered. The composition for soaking administration according to the present invention gives reduced burdens in administration as compared with conventional methods for inhibiting fish diseases, which involve fish-by-fish vaccine injection. In addition, the composition for soaking administration according to the present invention is not limited to specific fish species, and applicable to rearing of a wide variety of biological species.

The plantaricin, nisin, or lactic acid bacterial cell preparation content in the rearing water is not limited as long as the antibacterial activity to *Lactococcus garvieae* is allowed to be exhibited, and may be an amount suitable for the bacterial cell density of *Lactococcus garvieae,* the rearing place, the rearing temperature, the oxygen concentration, the rearing density, the type of the fish of interest, and so on. The total amount (content) of the lactic acid bacterial cell preparation in the rearing water may be, for example, 1 × 10² to 1 × 10¹⁰ cfu/mL, or 1 × 10³ to 1 × 10¹⁰ cfu/mL, or 1 × 10⁴ to 1 × 10¹⁰ cfu/mL. The colony-forming unit can be determined by an agar plate culture method. The total concentration of plantaricin, nisin, and the lactic acid bacterial cell preparation contained in the rearing water is not limited, and may be 0.1 to 100,000 ppm, or 1 to 100,000 ppm, or 10 to 100,000 ppm. The total amount of plantaricin, nisin, and the lactic acid bacterial cell preparation can be measured by weighing with a balance or the like. The concentration can be calculated from the mass ratio to the rearing water on the basis of the total amount determined.

The rearing water additive may be an additive that can be added to common rearing water for organisms of fish, without limitation. The rearing water additive may be liquid or powder, and may be freeze-dried. The rearing water additive may be plantaricin, nisin, or the lactic acid bacterial cell preparation as it is. It is preferable that the rearing water additive be added to rearing water in such a manner that the total amount of plantaricin, nisin, and the bacterial cell preparation contained in the rearing water falls within the range shown above.

The rearing water additive may be added to rearing water before the beginning of rearing of an organism of fish, or added to rearing water in which an organism of fish is reared. Examples of methods for adding the rearing water additive to rearing water include the aforementioned method of mixing water to rear an organism of fish and any of the compositions for rearing an organism of fish.

An appropriate period can be selected for soaking an organism of fish in the rearing water containing plantaricin, nisin, or the lactic acid bacterial cell preparation. An organism of fish may be reared in the rearing water containing plantaricin, nisin, or the lactic acid bacterial preparation continuously over the whole period to rear the organism of fish, or the fish may be soaked in the rearing water only for part of the period. The period to rear in the rearing water containing plantaricin, nisin, or the lactic acid bacterial cell preparation may be, for example, 1 month to 3 years. Rearing in the rearing water containing plantaricin, nisin, or the lactic acid bacterial cell preparation may be performed in cycles of continuing and suspending each for any period.

Each of the compositions for rearing an organism of fish may be a composition for injection administration (injection). The composition for injection administration can inhibit infections caused by *Lactococcus garvieae* in an organism of fish to which the composition has been administered.

The composition for injection administration may further contain, for example, any of buffers, isotonic agents, analgesic agents, antiseptic agents, antibacterial agents, and antioxidants. Examples of the buffers include phosphates, acetates, carbonates, and citrates. Examples of the isotonic agents include sodium chloride, glycerin, and D-mannitol. Examples of the analgesic agents include benzyl alcohol. Examples of agents for the purpose of antisepsis include thimerosal, p-hydroxybenzoates, phenoxyethanol, chlorobutanol, benzyl alcohol, phenethyl alcohol, dehydroacetic acid, sorbic acid, various antiseptic agents, antibiotics, and synthetic antibacterial agents. Examples of the antioxidants include sulfites and ascorbic acid. The composition for injection administration may contain any of photo-absorbing dyes to serve as an aid for storage and efficacy (e.g., riboflavin, adenine, adenosine), chelating agents and reductants for stabilization (e.g., vitamin C, citric acid), carbohydrates (e.g., sorbitol, lactose, mannitol, starch, sucrose, glucose, dextran), casein digests, and various vitamins.

The composition for injection administration can be obtained by adding plantaricin, nisin, or the lactic acid bacterial cell preparation to a proper buffer. In an example, the composition for injection administration can be obtained by adding plantaricin, nisin, or the lactic acid bacterial cell preparation to a conventional fish vaccine. The composition for injection administration may be the lactic acid bacterial cell preparation as it is.

The amount of plantaricin, nisin, or the lactic acid bacterial cell preparation contained in the composition for injection administration is not limited as long as the antibacterial activity to *Lactococcus garvieae* is allowed to be exhibited, and may be an amount suitable for the bacterial cell density of *Lactococcus garvieae,* the rearing place, the rearing temperature, the oxygen concentration, the rearing density, the type of the fish of interest, and so on. The total amount of the lactic acid bacterial cell preparation in the composition for injection administration may be, for example, 1 × 10³ to 1 × 10¹² cfu (colony-forming unit)/g, or 1 × 10⁴ to 1 × 10¹² cfu/g, or 1 × 10⁵ to 1 × 10¹² cfu/g. The total amount of plantaricin, nisin, and the lactic acid bacterial cell preparation in the composition for injection administration may be 0.001% by mass to 30% by mass, and preferably 0.01% by mass to 10% by mass to the total weight of the composition for injection administration.

The composition for injection administration may be administered to an organism of fish once or multiple times. For example, the composition for injection administration is intramuscularly or intraperitoneally administered to an organism of fish. The dose in one administration may be 0.05 mL to 3.0 mL. The time of administration may be the juvenile stage, young stage, or adult stage of the fish.

An embodiment of the present invention is use of at least one selected from plantaricin and nisin in production of a fish rearing composition. An embodiment of the present invention is use of bacterial cells or a cell culture product of a bacterium having at least one selected from a plantaricin gene and a nisin gene, or an extract thereof in production of a fish rearing composition.

### [Methods for rearing organism of fish]

A method for rearing an organism of fish according to an embodiment of the present invention includes administering at least one selected from plantaricin and nisin to an organism of fish. A method for rearing an organism of fish according to an embodiment of the present invention includes administering bacterial cells or a cell culture product of a bacterium having at least one selected from a plantaricin gene and a nisin gene, or an extract thereof to an organism of fish. Each administering may be oral administration, soaking administration, or injection administration.

A method for rearing an organism of fish according to an embodiment of the present invention includes allowing an organism of fish to ingest at least one selected from plantaricin and nisin. A method for rearing an organism of fish according to an embodiment of the present invention includes allowing an organism of fish to ingest bacterial cells or a cell culture product of a bacterium having at least one selected from a plantaricin gene and a nisin gene, or an extract thereof. Plantaricin, nisin, and the lactic acid bacterial cell preparation may be provided as any of the above compositions for rearing an organism of fish.

A method for rearing an organism of fish according to an embodiment of the present invention includes soaking an organism of fish in rearing water containing at least one selected from plantaricin and nisin. A method for rearing an organism of fish according to an embodiment of the present invention includes soaking an organism of fish in rearing water containing bacterial cells or a cell culture product of a bacterium having at least one selected from a plantaricin gene and a nisin gene, or an extract thereof. The rearing water containing plantaricin, nisin, and the lactic acid bacterial cell preparation may be provided as any of the above compositions for rearing an organism of fish.

In the methods for rearing an organism of fish, any of the compositions for rearing an organism of fish may be given according to the aforementioned method of mixing water to rear an organism of fish and the composition for rearing an organism of fish.

A method for rearing an organism of fish according to an embodiment of the present invention includes injecting at least one selected from plantaricin and nisin into an organism of fish. A method for rearing an organism of fish according to an embodiment of the present invention includes injecting bacterial cells or a cell culture product of a bacterium having at least one selected from a plantaricin gene and a nisin gene, or an extract thereof into an organism of fish. Plantaricin, nisin, and the lactic acid bacterial cell preparation may be provided as any of the above compositions for rearing an organism of fish.

An embodiment of the present invention is use of at least one selected from plantaricin and nisin in rearing an organism of fish. An embodiment of the present invention is use of bacterial cells or a cell culture product of a bacterium having at least one selected from a plantaricin gene and a nisin gene, or an extract thereof in rearing an organism of fish.

### [Compositions for preventing or treating disease caused by Lactococcus garvieae in fish]

A composition for preventing or treating a disease caused by *Lactococcus garvieae* (streptococci) in fish according to an embodiment of the present invention contains at least one selected from plantaricin and nisin. A composition for preventing or treating a disease caused by *Lactococcus garvieae* in fish according to an embodiment of the present invention contains bacterial cells or a cell culture product of a bacterium having at least one selected from a plantaricin gene and a nisin gene, or an extract thereof. Fish infections caused by *Lactococcus garvieae* can be prevented or treated by using plantaricin, nisin, and the lactic acid bacterial cell preparation. Herein, treatment includes mitigation of a symptom and improvement in and complete cure of a symptom. Each of the preventive or therapeutic compositions may be a medicament for animals.

Plantaricin, nisin, and the lactic acid bacterial cell preparation each exhibit antibacterial activity to *Lactococcus garvieae,* and are capable of inhibiting the growth of *Lactococcus garvieae* or killing *Lactococcus garvieae.* Plantaricin, nisin, and the lactic acid bacterial cell preparation each exhibit antibacterial activity not only to previous (type I) *Lactococcus garvieae,* but also to novel (type II) *Lactococcus garvieae.* Plantaricin, nisin, and the lactic acid bacterial cell preparation each have direct antibacterial activity to causal bacteria for infections, with the mechanism for inhibiting infections being independent of hosts, and thus are capable of preventing fish infections in a more universal manner, irrespective of the type or growth stage of the fish of interest. Use of plantaricin, nisin, or the lactic acid bacterial cell preparation enables more stable aquaculture of organisms of fish.

Each of the preventive or therapeutic compositions may be a composition for oral administration, and may be administered in the form of a feed or feed additive to an organism of fish. The preventive or therapeutic composition may contain a component that can be contained in the composition for oral administration to be used for rearing an organism of fish. The preventive or therapeutic composition may further contain a known component having antibacterial activity to *Lactococcus garvieae* or component that increases the immunoreactivity of fish. The same shapes, production methods, and administration methods as for the composition for oral administration to be used for rearing an organism of fish may be applied to the preventive or therapeutic composition. The plantaricin, nisin, or lactic acid bacterial cell preparation content of the preventive or therapeutic composition is not limited as long as the content is such that infections caused by *Lactococcus garvieae* can be prevented or treated in an organism of fish to which the composition has been administered, and may be in the same content range as in the composition for oral administration to be used for rearing an organism of fish.

Each of the preventive or therapeutic compositions may be a composition for soaking administration, and may be in the form of rearing water or a rearing water additive. The preventive or therapeutic composition as rearing water or a rearing water additive may be used or added in the same manner as rearing water or rearing water additives to be used for rearing organisms of fish. The plantaricin, nisin, or lactic acid bacterial cell preparation content of the preventive or therapeutic composition is not limited as long as the content is such that infections caused by *Lactococcus garvieae* can be prevented or treated in an organism of fish which has been soaked in the composition, and may be in the same content range as in the rearing water to be used for rearing an organism of fish.

Each of the preventive or therapeutic compositions may be a composition for injection administration (injection). The preventive or therapeutic composition may contain a component that can be contained in the composition for injection administration to be used for rearing an organisms of fish. The preventive or therapeutic composition may further contain a known component having antibacterial activity to *Lactococcus garvieae* or component that increases the immunoreactivity of fish. The same shapes, production methods, and administration methods as for the composition for injection administration to be used for rearing an organism of fish may be applied to the preventive or therapeutic composition. The plantaricin, nisin, or lactic acid bacterial cell preparation content of the preventive or therapeutic composition is not limited as long as the content is such that infections caused by *Lactococcus garvieae* can be prevented or treated in an organism of fish to which the composition has been administered by injection, and may be in the same content range as in the composition for injection administration to be used for rearing an organism of fish.

An embodiment of the present invention is use of at least one selected from plantaricin and nisin in production of a composition for preventing or treating a fish infection caused by *Lactococcus garvieae.* An embodiment of the present invention is use of bacterial cells or a cell culture product of a bacterium having at least one selected from a plantaricin gene and a nisin gene, or an extract thereof in production of a composition for preventing or treating a fish infection caused by *Lactococcus garvieae.*

### [Methods for preventing or treating disease caused by Lactococcus garvieae in fish]

A method for preventing or treating a disease caused by *Lactococcus garvieae* in fish according to an embodiment of the present invention includes administering at least one selected from plantaricin and nisin to an organism of fish. A method for preventing or treating a disease caused by *Lactococcus garvieae* in fish according to an embodiment of the present invention includes administering bacterial cells or a cell culture product of a bacterium having at least one selected from a plantaricin gene and a nisin gene, or an extract thereof to an organism of fish. Each administrating may be oral administration, soaking administration, or injection administration.

A method for preventing or treating a disease caused by *Lactococcus garvieae* in fish according to an embodiment of the present invention includes allowing an organism of fish to ingest at least one selected from plantaricin and nisin. A method for preventing or treating a disease caused by *Lactococcus garvieae* in fish according to an embodiment of the present invention includes allowing an organism of fish to ingest bacterial cells or a cell culture product of a bacterium having at least one selected from a plantaricin gene and a nisin gene, or an extract thereof. Plantaricin, nisin, and the lactic acid bacterial cell preparation may be provided as any of the preventive or therapeutic compositions to an organism of fish.

A method for preventing or treating a disease caused by *Lactococcus garvieae* in fish according to an embodiment of the present invention includes soaking an organism of fish in rearing water containing at least one selected from plantaricin and nisin. A method for preventing or treating a disease caused by *Lactococcus garvieae* in fish according to an embodiment of the present invention includes soaking an organism of fish in rearing water containing bacterial cells or a cell culture product of a bacterium having at least one selected from a plantaricin gene and a nisin gene, or an extract thereof. The rearing water containing plantaricin, nisin, and the lactic acid bacterial cell preparation may be provided as any of the preventive or therapeutic compositions.

A method for preventing or treating a disease caused by *Lactococcus garvieae* in fish according to an embodiment of the present invention includes injecting at least one selected from plantaricin and nisin into an organism of fish. A method for preventing or treating a disease caused by *Lactococcus garvieae* in fish according to an embodiment of the present invention includes injecting bacterial cells or a cell culture product of a bacterium having at least one selected from a plantaricin gene and a nisin gene, or an extract thereof into an organism of fish. Plantaricin, nisin, and the lactic acid bacterial cell preparation may be provided as any of the preventive or therapeutic compositions to an organism of fish.

An embodiment of the present invention is use of at least one selected from plantaricin and nisin for preventing or treating a disease caused by *Lactococcus garvieae* in fish. An embodiment of the present invention is use of bacterial cells or a cell culture product of a bacterium having at least one selected from a plantaricin gene and a nisin gene, or an extract thereof for preventing or treating a disease caused by *Lactococcus garvieae* in fish.

### [Growth inhibitor or bactericide, and growth-inhibiting or bactericidal method for Lactococcus garvieae]

A growth inhibitor or a bactericide for *Lactococcus garvieae* according to an embodiment of the present invention contains at least one selected from plantaricin and nisin. A growth inhibitor or a bactericide for *Lactococcus garvieae* according to an embodiment of the present invention contains bacterial cells or a cell culture product of a bacterium having at least one selected from a plantaricin gene and a nisin gene, or an extract thereof. Plantaricin, nisin, and the lactic acid bacterial cell preparation are capable of inhibiting the growth of *Lactococcus garvieae,* and killing *Lactococcus garvieae.* The growth inhibitor or the bactericide may be in the same form as the compositions for rearing an organism of fish. The growth inhibitor or the bactericide may further contain a substance having antibacterial activity to *Lactococcus garvieae.*

The lactic acid bacterial cell preparation content of the growth inhibitor or the bactericide for *Lactococcus garvieae* is not limited, and may be, for example, 1 × 10³ to 1 × 10¹² cfu/mL, or 1 × 10⁵ to 1 × 10¹² cfu/mL, or 1 × 10⁷ to 1 × 10¹² cfu/mL. The total concentration of plantaricin, nisin, and the lactic acid bacterial cell preparation contained in the growth inhibitor or the bactericide for *Lactococcus garvieae* is not limited, and may be 0.01 to 100,000 ppm, or 0.1 to 100,000 ppm, or 1 to 100,000 ppm.

A growth-inhibiting or bactericidal method for *Lactococcus garvieae* according to an embodiment of the present invention includes bringing at least one selected from plantaricin and nisin into contact with *Lactococcus garvieae.* A growth-inhibiting or bactericidal method for *Lactococcus garvieae* according to an embodiment of the present invention includes bringing bacterial cells or a cell culture product of a bacterium having at least one selected from a plantaricin gene and a nisin gene, or an extract thereof into contact with *Lactococcus garvieae.* Any method of bringing plantaricin, nisin, or the lactic acid bacterial cell preparation into contact with *Lactococcus garvieae* is applicable without limitation. For example, plantaricin, nisin, or the lactic acid bacterial cell preparation may be added to water in which *Lactococcus garvieae* is present, and an object with the presence of *Lactococcus garvieae* may be soaked in a liquid containing plantaricin, nisin, or the lactic acid bacterial cell preparation. A host infected with *Lactococcus garvieae* may be allowed to ingest a composition containing plantaricin, nisin, or the lactic acid bacterial cell preparation, and a host infected with *Lactococcus garvieae* may be soaked in rearing water containing plantaricin, nisin, or the lactic acid bacterial cell preparation. A composition containing plantaricin, nisin, or the lactic acid bacterial cell preparation may be injected into a host infected with *Lactococcus garvieae.* Plantaricin, nisin, and the lactic acid bacterial cell preparation may be used in the form of any of the growth inhibitor or the bactericide for *Lactococcus garvieae.*

An embodiment of the present invention is use of at least one selected from plantaricin and nisin in production of a growth inhibitor or a bactericide for *Lactococcus garvieae.* An embodiment of the present invention is use of bacterial cells or a cell culture product of a bacterium having at least one selected from a plantaricin gene and a nisin gene, or an extract thereof in production of a growth inhibitor or a bactericide for *Lactococcus garvieae.*

An embodiment of the present invention is use of at least one selected from plantaricin and nisin for inhibiting the growth of or killing *Lactococcus garvieae.* An embodiment of the present invention is use of bacterial cells or a cell culture product of a bacterium having at least one selected from a plantaricin gene and a nisin gene, or an extract thereof for inhibiting the growth of or killing *Lactococcus garvieae.*

### EXAMPLES

Hereinafter, the present invention will be described in more detail with reference to Examples, but the present invention is not limited to those Examples.

### [Experiment 1: Isolation and identification of NITE BP-03198]

A source for isolation (salted squid) was ground together with sterile water. The solution given by grinding was appropriately diluted and added to 1/2 MRS liquid medium, and subjected to enrichment culture. The enrichment culture solution was smeared on MRS agar medium containing calcium carbonate, and a microorganism that formed a halo was isolated. Hereinafter, this isolate is referred to as isolate A. No bubble was generated when the culture solution of isolate A was suspended in hydrogen peroxide. From the absence of catalase, isolate A was confirmed to be a lactic acid bacterium.

Isolate A was identified through 16S rRNA gene analysis, morphological observation, and physiological/biochemical characteristics test.

### (1) 16S rRNA gene analysis

Genomic DNA was extracted from isolate A, and PCR amplification of a 16S rRNA gene was performed by using the extracted genomic DNA as a template with a forward primer for cloning, 9F, and a reverse primer for cloning, 1510R (NAKAGAWA Yasuyoshi et al.: Gene Analysis Method - Nucleotide Sequence Determination Method for 16S rRNA Gene, edited by The Society for Actinomycetes Japan, Identification Manual of Actinomycetes, pp. 88-117, Business Center for Academic Societies Japan, 2001). Tks Gflex DNA polymerase (manufactured by Takara Bio Inc.) was used in the PCR amplification performed, and a PCR amplification product was purified.

Cycle sequence reaction was performed with the purified PCR amplification product. A BigDye Terminator v3.1 Cycle Sequencing Kit was used in the cycle sequence reaction performed. The resulting reaction solution was purified, and the purified solution was subjected to DNA sequence analysis (3130xl DNA Analyzer) to determine the nucleotide sequence of the 16S rRNA gene of the template DNA extracted from isolate A. Primers used for the sequence analysis were 9F, 515F, 1099F, 536R, 926R, and 1510R (NAKAGAWA Yasuyoshi et al.: Gene Analysis Method - Nucleotide Sequence Determination Method for 16S rRNA Gene, edited by The Society for Actinomycetes Japan, Identification Manual of Actinomycetes, pp. 88-117, Business Center for Academic Societies Japan, 2001).

With the microorganism identification system "ENKI" (manufactured by TechnoSuruga Laboratory Co., Ltd.), the nucleotide sequence of the 16S rRNA gene of isolate A was subjected to BLAST homology search for the microorganism identification database DB-BA15.0 (established by TechnoSuruga Laboratory Co., Ltd.) and international nucleotide sequence databases (DDBJ/ENA(EMBL)/GenBank). The nucleotide sequence of the 16S rRNA gene of isolate A exhibited 99.87% identity with the nucleotide sequence of the 16S rRNA gene of *Lactobacillus pentosus* (JCM1558), 99.87% identity with the nucleotide sequence of the 16S rRNA gene of *Lactobacillus plantarum* subsp. *plantarum* (JCM1149), and 99.73% identity with the nucleotide sequence of the 16S rRNA gene of *Lactobacillus paraplantarum* (DSM10667). However, there was no microorganism having a 16S rRNA gene the nucleotide sequence of which completely matched with that of the 16S rRNA gene of isolate A.

### (2) Morphological observation and physiological/biochemical characteristics test

Isolated bacterium A was applied onto MRS agar medium, and subjected to aerobic culture at a temperature of 30°C for 48 hours, and the cell morphology, Gram stainability, mobility, and colony morphology were observed by the following methods. The cell morphology was observed with the optical microscope BX50F4 (manufactured by Olympus Corporation). In the Gram staining, Favor G "NISSUI" (manufactured by Nissui Pharmaceutical Co., Ltd.) was used. The colony morphology was observed with the stereomicroscope SMZ800N (manufactured by Nikon Corporation). According to methods described in Barrow & Feltham (Cowan and Steel's Manual for the Identification of Medical Bacteria, 3rd ed. Cambridge: Cambridge University Press; 1993.), test was performed on catalase reaction, oxidase reaction, acid/gas production from glucose, and oxidation/fermentation (O/F) of glucose. The physiological/biochemical characteristics reaction of the bacterium was examined by using an API50CHB kit (manufactured by bioMerieux, France).

As shown in Fig. 1(A), isolate A formed circular colonies. As shown in Fig. 1(B), isolate A was positive for Gram stainability. Table 1 and Table 2 show results of the physiological/biochemical characteristics test and fermentability test for isolate A. Isolate A was a Gram-positive bacillus without mobility, formed no spore, exhibited negativity for catalase reaction and oxidase reaction, and fermented glucose. These characteristics were consistent with those of the genus *Lactobacillus,* to which isolate A was expected to belong as shown by the results of the 16S rDNA partial nucleotide sequence analysis. Results of the fermentability test performed with an API kit showed that isolate A fermented galactose, fructose, melicitose, etc., and did not ferment glycerol, D-xylose, etc. Isolate A exhibited no arginine dihydrolase activity, and grew at 15°C. These characteristics were different from those of *L. pentosus* in that no fermentation of glycerol and D-xylose was caused and consistent with those of *L. plantarum,* wherein *L. pentosus* and *L. plantarum* were the species to either one of which isolate A was expected to belong as suggested by the results of the 16S rDNA partial nucleotide sequence analysis. Accordingly, isolate A was found to be a novel isolate belonging to *Lactobacillus plantarum.* Isolate A was internationally deposited as NITE BP-03198.

NITE BP-03198 had plantaricin E, F, J, K, NC8α, and NC8β genes, the products of which are antibacterial peptides. The DNA sequence of the plantaricin E gene possessed by NITE BP-03198 and the corresponding peptide sequence are shown in SEQ ID NOs: 8 and 9, and the DNA sequence of the plantaricin F gene possessed by NITE BP-03198 and the corresponding peptide sequence are shown in SEQ ID NOs: 10 and 11. The DNA sequence of the plantaricin J gene possessed by NITE BP-03198 and the corresponding peptide sequence are shown in SEQ ID NOs: 12 and 13, and the DNA sequence of the plantaricin K gene possessed by NITE BP-03198 and the corresponding peptide sequence are shown in SEQ ID NOs: 14 and 15. The DNA sequence of the plantaricin NC8α gene possessed by NITE BP-03198 and the corresponding peptide sequence are shown in SEQ ID NOs: 16 and 17, and the DNA sequence of the plantaricin NC8β gene possessed by NITE BP-03198 and the corresponding peptide sequence are shown in SEQ ID NOs: 18 and 19.

**[Table 1]**

| | | |
|---|---|---|
| Test item | | NITE BP-03198 |
| Culture temperature | | 30°C |
| Cell morphology | | bacillus (0.6-0.7×1.0-3.0 µm) |
| Gram stainability | | + |
| Presence or absence of spore | | - |
| Mobility | | - |
| Colony morphology | Medium | MRS agar medium |
| | Culture time | 48 hours |
| | Diameter | 1 to 2 mm |
| | Color | milky white |
| | Shape | circular |
| | Elevation | lens-like |
| | Margin | entire |
| | Surface shape, etc. | smooth |
| | Transparency | opaque |
| | Viscosity | butter-like |
| Growth temperature test | 15°C | + |
| | 37°C | + |
| | 45°C | - |
| Arginine dihydrolase activity | | |
| Catalase reaction | | - |
| Oxidase reaction | | - |
| Acid/gas production from glucose (acid production/gas production) | | + / - |
| O/F test (oxidation/fermentation) | | + / + |

| | | |
|---|---|---|
| +: positive, -: negative | | |

**[Table 2]**

| Item | Substrate component | Test result | Item | Substrate component | Test result |
|---|---|---|---|---|---|
| 0 | Control | - | 25 | Esculin | + |
| 1 | Glycerol | - | 26 | Salicin | + |
| 2 | Erythritol | - | 27 | Cellobiose | + |
| 3 | D-Arabinose | - | 28 | Maltose | + |
| 4 | L-Arabinose | + | 29 | Lactose | + |
| 5 | Ribose | + | 30 | Melibiose | + |
| 6 | D-Xylose | - | 31 | Sucrose | + |
| 7 | L-Xylose | - | 32 | Trehalose | + |
| 8 | Adonitol | - | 33 | Inulin | - |
| 9 | β-Methyl-D-xylose | - | 34 | Melicitose | + |
| 10 | Galactose | + | 35 | Raffinose | + |
| 11 | Glucose | + | 36 | Starch | - |
| 12 | Fructose | + | 37 | Glycogen | - |
| 13 | Mannose | + | 38 | Xylitol | - |
| 14 | Sorbose | - | 39 | Gentiobiose | + |
| 15 | Rhamnose | - | 40 | D-Turanose | + |
| 16 | Dulcitol | - | 41 | D-Lyxose | - |
| 17 | Inositol | - | 42 | D-Tagatose | - |
| 18 | Mannitol | + | 43 | D-Fucose | - |
| 19 | Sorbitol | + | 44 | L-Fucose | - |
| 20 | α-Methyl-D-mannoside | + | 45 | D-Arabitol | - |
| 21 | α-Methyl-D-glucoside | - | 46 | L-Arabitol | - |
| 22 | N-Acetylglucosamine | + | 47 | Gluconate | + |
| 23 | Amygdalin | + | 48 | 2-Ketogluconate | - |
| 24 | Arbutin | + | 49 | 5-Ketogluconate | - |

| | | | | | |
|---|---|---|---|---|---|
| +: positive, -: negative | | | | | |

### [Experiment 2: Isolation and identification of NITE BP-03199]

Isolate B was isolated in the same manner as in Experiment 1 except that wax apple was used as a source for isolation. Identification of isolate B was performed in the same manner as in Experiment 1.

The nucleotide sequence of the 16S rRNA gene of isolate B exhibited 100.0% identity with the nucleotide sequence of the 16S rRNA gene of *Lactobacillus pentosus* (JCM1558), 100.0% identity with the nucleotide sequence of the 16S rRNA gene of *Lactobacillus plantarum* subsp. *plantarum* (JCM1149), and 99.80% identity with the nucleotide sequence of the 16S rRNA gene of *Lactobacillus paraplantarum* (DSM10667).

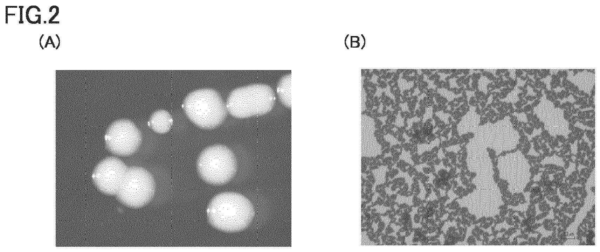

As shown in Fig. 2(A), isolate B formed circular colonies. As shown in Fig. 2(B), isolate B was positive for Gram stainability. Table 3 and Table 4 show results of the physiological/biochemical characteristics test and fermentability test for isolate B. Isolate B was a Gram-positive bacillus without mobility, formed no spore, exhibited negativity for catalase reaction and oxidase reaction, and fermented glucose. These characteristics were consistent with those of the genus *Lactobacillus,* to which isolate B was expected to belong as shown by the results of the 16S rDNA partial nucleotide sequence analysis. Results of the fermentability test performed with an API kit showed that isolate B fermented galactose, fructose, α-methyl-D-mannoside, melicitose, etc., and did not ferment glycerol, D-xylose, etc. Isolate B exhibited no arginine dihydrolase activity, and grew at 15°C. These characteristics were different from those of *L. pentosus* in that no fermentation of glycerol and D-xylose was caused and consistent with those of *L. plantarum,* wherein *L. pentosus* and *L. plantarum* were the species to either one of which isolate B was expected to belong as suggested by the results of the 16S rDNA partial nucleotide sequence analysis. Accordingly, isolate B was found to be a novel isolate belonging to *Lactobacillus plantarum.* Isolate B was internationally deposited as NITE BP-03199.

NITE BP-03199 had plantaricin A, E, F, J, and K genes, the products of which are antibacterial peptides. The DNA sequence of the plantaricin A gene possessed by NITE BP-03199 and the corresponding peptide sequence are shown in SEQ ID NOs: 20 and 21, and the DNA sequence of the plantaricin E gene possessed by NITE BP-03199 and the corresponding peptide sequence are shown in SEQ ID NOs: 22 and 23. The DNA sequence of the plantaricin F gene possessed by NITE BP-03199 and the corresponding peptide sequence are shown in SEQ ID NOs: 24 and 25, and the DNA sequence of the plantaricin J gene possessed by NITE BP-03199 and the corresponding peptide sequence are shown in SEQ ID NOs: 26 and 27. The DNA sequence of the plantaricin K gene possessed by NITE BP-03199 and the corresponding peptide sequence are shown in SEQ ID NOs: 28 and 29.

**[Table 3]**

| | | |
|---|---|---|
| Test item | | NITE BP-03199 |
| Culture temperature | | 30°C |
| Cell morphology | | bacillus (0.7-0.8×2.0-3.0 µm) |
| Gram stainability | | + |
| Presence or absence of spore | | - |
| Mobility | | - |
| Colony morphology | Medium | MRS agar medium |
| | Culture time | 48 hours |
| | Diameter | 1 to 3 mm |
| | Color | milky white |
| | Shape | circular |
| | Elevation | lens-like |
| | Margin | entire |
| | Surface shape, etc. | smooth |
| | Transparency | opaque |
| | Viscosity | butter-like |
| Growth temperature test | 15°C | + |
| | 37°C | + |
| | 45°C | - |
| Arginine dihydrolase activity | | - |
| Catalase reaction | | - |
| Oxidase reaction | | - |
| Acid/gas production from glucose (acid production/gas production) | | + / - |
| O/F test (oxidation/fermentation) | | + / + |

| | | |
|---|---|---|
| +: positive, -: negative | | |

**[Table 4]**

| Item | Substrate component | Test result | Item | Substrate component | Test result |
|---|---|---|---|---|---|
| 0 | Control | - | 25 | Esculin | + |
| 1 | Glycerol | - | 26 | Salicin | + |
| 2 | Erythritol | - | 27 | Cellobiose | + |
| 3 | D-Arabinose | - | 28 | Maltose | + |
| 4 | L-Arabinose | + | 29 | Lactose | + |
| 5 | Ribose | + | 30 | Melibiose | + |
| 6 | D-Xylose | - | 31 | Sucrose | + |
| 7 | L-Xylose | - | 32 | Trehalose | + |
| 8 | Adonitol | - | 33 | Inulin | - |
| 9 | β-Methyl-D-xylose | - | 34 | Melicitose | + |
| 10 | Galactose | + | 35 | Raffinose | + |
| 11 | Glucose | + | 36 | Starch | - |
| 12 | Fructose | + | 37 | Glycogen | - |
| 13 | Mannose | + | 38 | Xylitol | - |
| 14 | Sorbose | - | 39 | Gentiobiose | + |
| 15 | Rhamnose | - | 40 | D-Turanose | + |
| 16 | Dulcitol | - | 41 | D-Lyxose | - |
| 17 | Inositol | - | 42 | D-Tagatose | - |
| 18 | Mannitol | + | 43 | D-Fucose | - |
| 19 | Sorbitol | + | 44 | L-Fucose | - |
| 20 | α-Methyl-D-mannoside | + | 45 | D-Arabitol | - |
| 21 | α-Methyl-D-glucoside | + | 46 | L-Arabitol | - |
| 22 | N-Acetylglucosamine | + | 47 | Gluconate | + |
| 23 | Amygdalin | + | 48 | 2-Ketogluconate | - |
| 24 | Arbutin | + | 49 | 5-Ketogluconate | - |

| | | | | | |
|---|---|---|---|---|---|
| +: positive, -: negative | | | | | |

### [Experiment 3: Isolation and identification of NITE BP-03200]

Isolate C was isolated in the same manner as in Experiment 1 except that *Pandanus odoratissimus* was used as a source for isolation. Identification of isolate C was performed in the same manner as in Experiment 1.

The nucleotide sequence of the 16S rRNA gene of isolate C exhibited 99.87% identity with the nucleotide sequence of the 16S rRNA gene of *Lactobacillus pentosus* (JCM1558), 99.87% identity with the nucleotide sequence of the 16S rRNA gene of *Lactobacillus plantarum* subsp. *plantarum* (JCM1149), and 99.66% identity with the nucleotide sequence of the 16S rRNA gene of *Lactobacillus paraplantarum* (DSM10667). However, there was no microorganism having a 16S rRNA gene the nucleotide sequence of which completely matched with that of the 16S rRNA gene of isolate C.

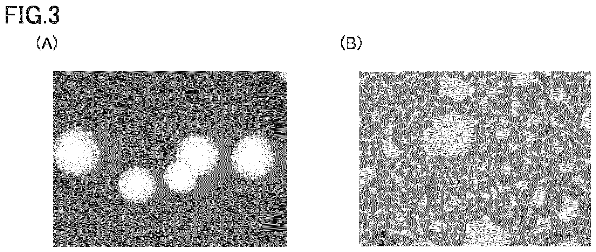

As shown in Fig. 3(A), isolate C formed circular colonies. As shown in Fig. 3(B), isolate C was positive for Gram stainability. Table 5 and Table 6 show results of the physiological/biochemical characteristics test and fermentability test for isolate C. Isolate C was a Gram-positive bacillus without mobility, formed no spore, exhibited negativity for catalase reaction and oxidase reaction, and fermented glucose. These characteristics were consistent with those of the genus *Lactobacillus,* to which isolate C was expected to belong as shown by the results of the 16S rDNA partial nucleotide sequence analysis. Results of the fermentability test performed with an API kit showed that isolate C fermented galactose, fructose, α-methyl-D-mannoside, melicitose, etc., and did not ferment glycerol, D-xylose, etc. Isolate C exhibited no arginine dihydrolase activity, and grew at 15°C. These characteristics were different from those of *L. pentosus* in that no fermentation of glycerol and D-xylose was caused and consistent with those of *L. plantarum,* wherein *L. pentosus* and *L. plantarum* were the species demonstrated to be relatives to isolate C by the results of the 16S rDNA partial nucleotide sequence analysis. Accordingly, isolate C was found to be a novel isolate belonging to *Lactobacillus plantarum.* Isolate C was internationally deposited as NITE BP-03200.

NITE BP-03200 had plantaricin A, E, F, NC8α, and NC8β genes, the products of which are antibacterial peptides. The DNA sequence of the plantaricin A gene possessed by NITE BP-03200 and the corresponding peptide sequence are shown in SEQ ID NOs: 30 and 31, and the DNA sequence of the plantaricin E gene possessed by NITE BP-03200 and the corresponding peptide sequence are shown in SEQ ID NOs: 32 and 33. The DNA sequence of the plantaricin F gene possessed by NITE BP-03200 and the corresponding peptide sequence are shown in SEQ ID NOs: 34 and 35, and the DNA sequence of the plantaricin NC8α gene possessed by NITE BP-03200 and the corresponding peptide sequence are shown in SEQ ID NOs: 36 and 37. The DNA sequence of the plantaricin NC8β gene possessed by NITE BP-03200 and the corresponding peptide sequence are shown in SEQ ID NOs: 38 and 39.

**[Table 5]**

| | | |
|---|---|---|
| Test item | | NITE BP-03200 |
| Culture temperature | | 30°C |
| Cell morphology | | bacillus (0.7-0.8×2.0-3.0 µm) |
| Gram stainability | | + |
| Presence or absence of spore | | - |
| Mobility | | - |
| Colony morphology | Medium | MRS agar medium |
| | Culture time | 48 hours |
| | Diameter | 1 to 3 mm |
| | Color | milky white |
| | Shape | circular |
| | Elevation | lens-like |
| | Margin | entire |
| | Surface shape, etc. | smooth |
| | Transparency | opaque |
| | Viscosity | butter-like |
| Growth temperature test | 15°C | + |
| | 37°C | + |
| | 45°C | - |
| Arginine dihydrolase activity | | - |
| Catalase reaction | | - |
| Oxidase reaction | | - |
| Acid/gas production from glucose (acid production/gas production) | | + / - |
| O/F test (oxidation/fermentation) | | + / + |

| | | |
|---|---|---|
| +: positive, -: negative | | |

**[Table 6]**

| Item | Substrate component | Test result | Item | Substrate component | Test result |
|---|---|---|---|---|---|
| 0 | Control | - | 25 | Esculin | + |
| 1 | Glycerol | - | 26 | Salicin | + |
| 2 | Erythritol | - | 27 | Cellobiose | + |
| 3 | D-Arabinose | - | 28 | Maltose | + |
| 4 | L-Arabinose | - | 29 | Lactose | + |
| 5 | Ribose | + | 30 | Melibiose | + |
| 6 | D-Xylose | - | 31 | Sucrose | + |
| 7 | L-Xylose | - | 32 | Trehalose | + |
| 8 | Adonitol | - | 33 | Inulin | - |
| 9 | β-Methyl-D-xylose | - | 34 | Melicitose | + |
| 10 | Galactose | + | 35 | Raffinose | + |
| 11 | Glucose | + | 36 | Starch | - |
| 12 | Fructose | + | 37 | Glycogen | - |
| 13 | Mannose | + | 38 | Xylitol | - |
| 14 | Sorbose | - | 39 | Gentiobiose | + |
| 15 | Rhamnose | - | 40 | D-Turanose | + |
| 16 | Dulcitol | - | 41 | D-Lyxose | - |
| 17 | Inositol | - | 42 | D-Tagatose | - |
| 18 | Mannitol | + | 43 | D-Fucose | - |
| 19 | Sorbitol | + | 44 | L-Fucose | - |
| 20 | α-Methyl-D-mannoside | + | 45 | D-Arabitol | - |
| 21 | α-Methyl-D-glucoside | - | 46 | L-Arabitol | - |
| 22 | N-Acetylglucosamine | + | 47 | Gluconate | + |
| 23 | Amygdalin | + | 48 | 2-Ketogluconate | - |
| 24 | Arbutin | + | 49 | 5-Ketogluconate | - |

| | | | | | |
|---|---|---|---|---|---|
| +: positive, -: negative | | | | | |

### [Experiment 4: Isolation and identification of NITE BP-03201]

Isolate D was isolated in the same manner as in Experiment 1 except that *Ficus variegata Blume* was used as a source for isolation. Identification of isolate D was performed in the same manner as in Experiment 1.

The nucleotide sequence of the 16S rRNA gene of isolate D exhibited 99.93% identity with the nucleotide sequence of the 16S rRNA gene of *Lactobacillus pentosus* (JCM1558), 99.93% identity with the nucleotide sequence of the 16S rRNA gene of *Lactobacillus plantarum* subsp. *plantarum* (JCM1149), and 99.73% identity with the nucleotide sequence of the 16S rRNA gene of *Lactobacillus paraplantarum* (DSM10667). However, there was no microorganism having a 16S rRNA gene the nucleotide sequence of which completely matched with that of the 16S rRNA gene of isolate D.

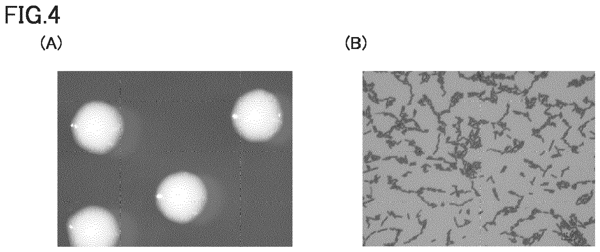

As shown in Fig. 4(A), isolate D formed circular colonies. As shown in Fig. 4(B), isolate D was positive for Gram stainability. Table 7 and Table 8 show results of the physiological/biochemical characteristics test and fermentability test for isolate D. Isolate D was a Gram-positive bacillus without mobility, formed no spore, exhibited negativity for catalase reaction and oxidase reaction, and fermented glucose. These characteristics were consistent with those of the genus *Lactobacillus,* to which isolate D was expected to belong as shown by the results of the 16S rDNA partial nucleotide sequence analysis. Results of the fermentability test performed with an API kit showed that isolate D fermented galactose, fructose, melicitose, etc., and did not ferment glycerol, D-xylose, etc. Isolate D exhibited no arginine dihydrolase activity, and grew at 15°C. These characteristics were different from those of *L. pentosus* in that no fermentation of glycerol and D-xylose was caused and consistent with those of *L. plantarum,* wherein *L. pentosus* and *L. plantarum* were the species to either one of which isolate D was expected to belong as suggested by the results of the 16S rDNA partial nucleotide sequence analysis. Accordingly, isolate D was found to be a novel isolate belonging to *Lactobacillus plantarum.* Isolate D was internationally deposited as NITE BP-03201.

NITE BP-03201 had plantaricin A, E, F, J, and K genes, the products of which are antibacterial peptides. The DNA sequence of the plantaricin A gene possessed by NITE BP-03201 and the corresponding peptide sequence are shown in SEQ ID NOs: 40 and 41, and the DNA sequence of the plantaricin E gene possessed by NITE BP-03201 and the corresponding peptide sequence are shown in SEQ ID NOs: 42 and 43. The DNA sequence of the plantaricin F gene possessed by NITE BP-03201 and the corresponding peptide sequence are shown in SEQ ID NOs: 44 and 45, and the DNA sequence of the plantaricin J gene possessed by NITE BP-03201 and the corresponding peptide sequence are shown in SEQ ID NOs: 46 and 47. The DNA sequence of the plantaricin K gene possessed by NITE BP-03201 and the corresponding peptide sequence are shown in SEQ ID NOs: 48 and 49.

**[Table 7]**

| | | |
|---|---|---|
| Test item | | NITE BP-03201 |
| Culture temperature | | 30°C |
| Cell morphology | | bacillus (0.9-1.0×2.0-4.0 µm) |
| Gram stainability | | + |
| Presence or absence of spore | | - |
| Mobility | | - |
| Colony morphology | Medium | MRS agar medium |
| | Culture time | 48 hours |
| | Diameter | 1 to 3 mm |
| | Color | milky white |
| | Shape | circular |
| | Elevation | lens-like |
| | Margin | entire |
| | Surface shape, etc. | smooth |
| | Transparency | opaque |
| | Viscosity | butter-like |
| Growth temperature test | 15°C | + |
| | 37°C | + |
| | 45°C | - |
| Arginine dihydrolase activity | | - |
| Catalase reaction | | - |
| Oxidase reaction | | - |
| Acid/gas production from glucose (acid production/gas production) | | + / - |
| O/F test (oxidation/fermentation) | | + / + |

| | | |
|---|---|---|
| +: positive, -: negative | | |

**[Table 8]**

| Item | Substrate component | Test result | Item | Substrate component | Test result |
|---|---|---|---|---|---|
| 0 | Control | - | 25 | Esculin | + |
| 1 | Glycerol | - | 26 | Salicin | + |
| 2 | Erythritol | - | 27 | Cellobiose | + |
| 3 | D-Arabinose | - | 28 | Maltose | + |
| 4 | L-Arabinose | + | 29 | Lactose | + |
| 5 | Ribose | + | 30 | Melibiose | + |
| 6 | D-Xylose | - | 31 | Sucrose | + |
| 7 | L-Xylose | - | 32 | Trehalose | + |
| 8 | Adonitol | - | 33 | Inulin | - |
| 9 | β-Methyl-D-xylose | - | 34 | Melicitose | + |
| 10 | Galactose | + | 35 | Raffinose | + |
| 11 | Glucose | + | 36 | Starch | - |
| 12 | Fructose | + | 37 | Glycogen | - |
| 13 | Mannose | + | 38 | Xylitol | - |
| 14 | Sorbose | - | 39 | Gentiobiose | + |
| 15 | Rhamnose | - | 40 | D-Turanose | + |
| 16 | Dulcitol | - | 41 | D-Lyxose | - |
| 17 | Inositol | - | 42 | D-Tagatose | - |
| 18 | Mannitol | + | 43 | D-Fucose | - |
| 19 | Sorbitol | + | 44 | L-Fucose | - |
| 20 | α-Methyl-D-mannoside | - | 45 | D-Arabitol | - |
| 21 | α-Methyl-D-glucoside | - | 46 | L-Arabitol | - |
| 22 | N-Acetylglucosamine | + | 47 | Gluconate | + |
| 23 | Amygdalin | + | 48 | 2-Ketogluconate | - |
| 24 | Arbutin | + | 49 | 5-Ketogluconate | - |

| | | | | | |
|---|---|---|---|---|---|
| +: positive, -: negative | | | | | |

### [Experiment 5: Isolation and identification of NITE BP-03202]

Isolate E was isolated in the same manner as in Experiment 1 except that pine cones were used as a source for isolation. Identification of isolate E was performed in the same manner as in Experiment 1.

The nucleotide sequence of the 16S rRNA gene of isolate E exhibited 99.93% identity with the nucleotide sequence of the 16S rRNA gene of *Lactobacillus pentosus* (JCM1558), 99.93% identity with the nucleotide sequence of the 16S rRNA gene of *Lactobacillus plantarum* subsp. *plantarum* (JCM1149), and 99.73% identity with the nucleotide sequence of the 16S rRNA gene of *Lactobacillus paraplantarum* (DSM10667). However, there was no microorganism having a 16S rRNA gene the nucleotide sequence of which completely matched with that of the 16S rRNA gene of isolate E.

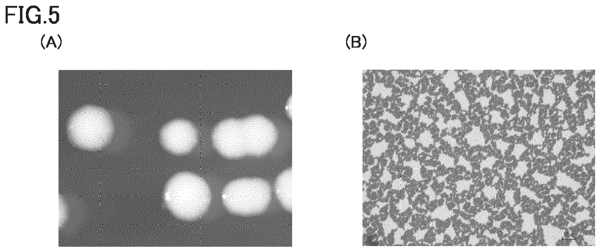

As shown in Fig. 5(A), isolate E formed circular colonies. As shown in Fig. 5(B), isolate E was positive for Gram stainability. Table 9 and Table 10 show results of the physiological/biochemical characteristics test and fermentability test for isolate E. Isolate E was a Gram-positive bacillus without mobility, formed no spore, exhibited negativity for catalase reaction and oxidase reaction, and fermented glucose. These characteristics were consistent with those of the genus *Lactobacillus,* to which isolate E was expected to belong as shown by the results of the 16S rDNA partial nucleotide sequence analysis. Results of the fermentability test performed with an API kit showed that isolate E fermented galactose, fructose, α-methyl-D-mannoside, melicitose, etc., and did not ferment glycerol, D-xylose, etc. Isolate E exhibited no arginine dihydrolase activity, and grew at 15°C. These characteristics were different from those of *L. pentosus* in that no fermentation of glycerol and D-xylose was caused and consistent with those of *L. plantarum,* wherein *L. pentosus* and *L. plantarum* were the species to either one of which isolate E was expected to belong as suggested by the results of the 16S rDNA partial nucleotide sequence analysis. Accordingly, isolate E was found to be a novel isolate belonging to *Lactobacillus plantarum.* Isolate E was internationally deposited as NITE BP-03202.

NITE BP-03202 had plantaricin E, F, J, K, NC8α, and NC8β genes, the products of which are antibacterial peptides. The DNA sequence of the plantaricin E gene possessed by NITE BP-03202 and the corresponding peptide sequence are shown in SEQ ID NOs: 50 and 51, and the DNA sequence of the plantaricin F gene possessed by NITE BP-03202 and the corresponding peptide sequence are shown in SEQ ID NOs: 52 and 53. The DNA sequence of the plantaricin J gene possessed by NITE BP-03202 and the corresponding peptide sequence are shown in SEQ ID NOs: 54 and 55, and the DNA sequence of the plantaricin K gene possessed by NITE BP-03202 and the corresponding peptide sequence are shown in SEQ ID NOs: 56 and 57. The DNA sequence of the plantaricin NC8α gene possessed by NITE BP-03202 and the corresponding peptide sequence are shown in SEQ ID NOs: 58 and 59, and the DNA sequence of the plantaricin NC8β gene possessed by NITE BP-03202 and the corresponding peptide sequence are shown in SEQ ID NOs: 60 and 61.

**[Table 9]**

| | | |
|---|---|---|
| Test item | | NITE BP-03202 |
| Culture temperature | | 30°C |
| Cell morphology | | bacillus (0.8-0.9×1.0-2.0 µm) |
| Gram stainability | | + |
| Presence or absence of spore | | - |
| Mobility | | - |
| Colony morphology | Medium | MRS agar medium |
| | Culture time | 48 hours |
| | Diameter | 1 to 2 mm |
| | Color | milky white |
| | Shape | circular |
| | Elevation | lens-like |
| | Margin | entire |
| | Surface shape, etc. | smooth |
| | Transparency | opaque |
| | Viscosity | butter-like |
| Growth temperature test | 15°C | + |
| | 37°C | + |
| | 45°C | - |
| Arginine dihydrolase activity | | - |
| Catalase reaction | | - |
| Oxidase reaction | | - |
| Acid/gas production from glucose (acid production/gas production) | | + / - |
| O/F test (oxidation/fermentation) | | + / + |

| | | |
|---|---|---|
| +: positive, -: negative | | |

**[Table 10]**

| Item | Substrate component | Test result | Item | Substrate component | Test result |
|---|---|---|---|---|---|
| 0 | Control | - | 25 | Esculin | + |
| 1 | Glycerol | - | 26 | Salicin | + |
| 2 | Erythritol | - | 27 | Cellobiose | + |
| 3 | D-Arabinose | - | 28 | Maltose | + |
| 4 | L-Arabinose | + | 29 | Lactose | + |
| 5 | Ribose | + | 30 | Melibiose | + |
| 6 | D-Xylose | - | 31 | Sucrose | + |
| 7 | L-Xylose | - | 32 | Trehalose | + |
| 8 | Adonitol | - | 33 | Inulin | - |
| 9 | β-Methyl-D-xylose | - | 34 | Melicitose | + |
| 10 | Galactose | + | 35 | Raffinose | + |
| 11 | Glucose | + | 36 | Starch | - |
| 12 | Fructose | + | 37 | Glycogen | - |
| 13 | Mannose | + | 38 | Xylitol | - |
| 14 | Sorbose | - | 39 | Gentiobiose | + |
| 15 | Rhamnose | - | 40 | D-Turanose | + |
| 16 | Dulcitol | - | 41 | D-Lyxose | - |
| 17 | Inositol | - | 42 | D-Tagatose | - |
| 18 | Mannitol | + | 43 | D-Fucose | - |
| 19 | Sorbitol | + | 44 | L-Fucose | - |
| 20 | α-Methyl-D-mannoside | + | 45 | D-Arabitol | - |
| 21 | α-Methyl-D-glucoside | + | 46 | L-Arabitol | - |
| 22 | N-Acetylglucosamine | + | 47 | Gluconate | + |
| 23 | Amygdalin | + | 48 | 2-Ketogluconate | - |
| 24 | Arbutin | + | 49 | 5-Ketogluconate | - |

| | | | | | |
|---|---|---|---|---|---|
| +: positive, -: negative | | | | | |

### [Experiment 6: Isolation and identification of NITE ABP-03481]

Isolate F was isolated in the same manner as in Experiment 1 except that *Ficus variegata Blume* was used as a source for isolation. Identification of isolate F was performed in the same manner as in Experiment 1.

The nucleotide sequence of the 16S rRNA gene of isolate F exhibited 100% identity with the nucleotide sequence of the 16S rRNA gene of *Lactococcus lactis* subsp. *lactis* (NCDO604 strain), and 99.9% identity with the nucleotide sequence of the 16S rRNA gene of *Lactococcus lactis* subsp. *hordniae* (NCDO2181 strain).

As shown in Fig. 6(A), isolate F formed circular colonies. As shown in Fig. 6(B), isolate F was positive for Gram stainability. Table 11 and Table 12 show results of the physiological/biochemical characteristics test and fermentability test for isolate F. Isolate F was a Gram-positive oval coccus without mobility, formed no spore, exhibited negativity for catalase reaction and oxidase reaction, and fermented glucose. These characteristics were consistent with those of the genus *Lactococcus,* to which isolate F was expected to belong as shown by the results of the 16S rDNA partial nucleotide sequence analysis. Results of the fermentability test performed with an API kit showed that isolate F fermented ribose, galactose, maltose, etc., and did not ferment melibiose, melicitose, etc. Isolate F grew at 15°C, grew in the presence of 4% NaCl, and exhibited arginine dihydrolase activity. These characteristics were consistent with those of *Lactococcus lactis* subsp. *lactis* of the two *Lactococcus lactis* subspecies, to either one of which isolate F was expected to belong as suggested by the results of the 16S rDNA partial nucleotide sequence analysis. In particular, the characteristics were different from those of *Lactococcus lactis* subsp. *hordniae* in that isolate F fermented ribose, maltose, etc., and grew with 4% NaCl. Accordingly, isolate F was found to be a novel isolate belonging to *Lactococcus lactis* subsp. *lactis.* Isolate F was internationally deposited as NITE ABP-03481.

NITE ABP-03481 had a nisin Z gene, the product of which is an antibacterial peptide. The DNA sequence of the nisin Z gene possessed by NITE ABP-03481 and the corresponding peptide sequence are shown in SEQ ID NOs: 62 and 63.

**[Table 11]**

| Test item | | NITE ABP-03481 |
|---|---|---|
| Culture temperature | | 30°C |
| Cell morphology | | oval coccus (0.5-0.6×0.6-0.9 µm) |
| Gram stainability | | + |
| Presence or absence of spore | | - |
| Mobility | | - |
| Colony morphology | Medium | MRS agar medium |
| | Culture time | 72 hours |
| | Diameter | 1.0 to 1.3 mm |
| | Color | milky white |
| | Shape | circular |
| | Elevation | lens-like |
| | Margin | entire |
| | Surface shape, etc. | smooth |
| | Transparency | opaque |
| | Viscosity | butter-like |
| Growth temperature test | 15°C | + |
| | 37°C | + |
| | 45°C | - |
| Growth with 2% NaCl | | + |
| Growth with 4% NaCl | | + |
| Arginine dihydrolase activity | | + |
| Catalase reaction | | - |
| Oxidase reaction | | - |
| Acid/gas production from glucose (acid production/gas production) | | + / - |
| O/F test (oxidation/fermentation) | | NT |

| | | |
|---|---|---|
| +: positive, -: negative, NT:Not Tested | | |

**[Table 12]**

| Item | Substrate component | Test result | Item | Substrate component | Test result |
|---|---|---|---|---|---|
| 0 | Control | - | 25 | Esculin | + |
| 1 | Glycerol | - | 26 | Salicin | + |
| 2 | Erythritol | - | 27 | Cellobiose | + |
| 3 | D-Arabinose | - | 28 | Maltose | + |
| 4 | L-Arabinose | + | 29 | Lactose | + |
| 5 | Ribose | + | 30 | Melibiose | - |
| 6 | D-Xylose | - | 31 | Sucrose | + |
| 7 | L-Xylose | - | 32 | Trehalose | + |
| 8 | Adonitol | - | 33 | Inulin | - |
| 9 | β-Methyl-D-xylose | - | 34 | Melicitose | - |
| 10 | Galactose | + | 35 | Raffinose | - |
| 11 | Glucose | + | 36 | Starch | + |
| 12 | Fructose | + | 37 | Glycogen | - |
| 13 | Mannose | + | 38 | Xylitol | - |
| 14 | Sorbose | - | 39 | Gentiobiose | + |
| 15 | Rhamnose | - | 40 | D-Turanose | - |
| 16 | Dulcitol | - | 41 | D-Lyxose | - |
| 17 | Inositol | - | 42 | D-Tagatose | - |
| 18 | Mannitol | + | 43 | D-Fucose | - |
| 19 | Sorbitol | - | 44 | L-Fucose | - |
| 20 | α-Methyl-D-mannoside | - | 45 | D-Arabitol | - |
| 21 | α-Methyl-D-glucoside | - | 46 | L-Arabitol | - |
| 22 | N-Acetylglucosamine | + | 47 | Gluconate | + |
| 23 | Amygdalin | + | 48 | 2-Ketogluconate | - |
| 24 | Arbutin | + | 49 | 5-Ketogluconate | - |

| | | | | | |
|---|---|---|---|---|---|
| +: positive, -: negative | | | | | |

### [Experiment 7: Test for evaluating antibacterial activities of lactic acid bacterial cell preparations]

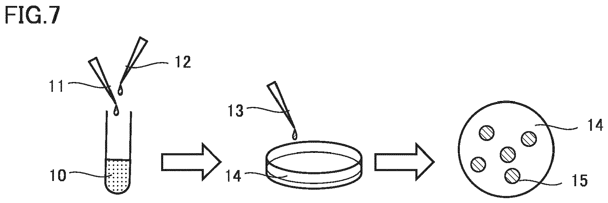

Examination was performed on whether bacterial cell preparations of NITE BP-03198, NITE BP-03199, NITE BP-03200, NITE BP-03202, ATCC-11454, and NITE ABP-03481 inhibit the growth of *Lactococcus garvieae* type I (TUMSAT-K9408 strain) and type II (TUMSAT-LgN1 strain). With reference to Fig. 7, the experimental method will be described. To 270 µL of Todd-Hewitt (TH) medium 10, 30 µL of a lactic acid bacterial cell preparation 11 was added. Furthermore, 3 µL of a bacterial solution 12 containing *Lactococcus garvieae* (approximately 10⁵ cfu/mL) was added, and shaking culture was performed at 25°C for 8 hours. To TH agar medium 14, 5 µL of a bacterial culture solution 13 was dropped, culture was performed at 25°C for 12 hours, and whether a colony 15 of *Lactococcus garvieae* formed was observed. If the number of colonies observed was five or less, it was determined that there was antibacterial activity. Lactic acid bacterial cell preparations were prepared in the following manner. For NITE BP-03198, NITE BP-03199, NITE BP-03200, and NITE BP-03202, first, the lactic acid bacteria were each cultured in MRS Broth at a temperature of 30°C, giving culture solutions. Thereafter, the culture solutions were centrifuged to precipitate bacterial cells, and the culture supernatants were collected. The culture supernatants collected were 10-fold concentrated by using an ultrafiltration membrane (Merck Millipore, AMICON ULTRA-15 3 KDa cutoff), giving bacterial cell preparations. For ATCC-11454 and NITE ABP-03481, bacterial cell preparations were obtained in the same manner as described for NITE BP-03198 and others. As a negative control, medium for lactic acid bacteria (MRS Broth) was added to a bacterial solution containing *Lactococcus garvieae.* Table 13 shows results on the types of plantaricin and nisin possessed by the lactic acid bacterial strains and the antibacterial activities to *Lactococcus garvieae.*

**[Table 13]**

| Bacterial strain | Plantaricin gene | Nisin gene | Antibacterial activity to *Lactococcus garvieae* | |
|---|---|---|---|---|
| | | | K9408 | LgN1 |
| NITE-BP-03198 | E,F,J,K,NC8α,NC8β | - | + | + |
| NITE-BP-03199 | A,E,F,J,K | - | + | + |
| NITE-BP-03200 | A,E,F,NC8α,NC8β | - | + | + |
| NITE-BP-03202 | E,F,J,K,NC8α,NC8β | - | + | + |
| ATCC-11454 | - | A | + | + |
| NITE-ABP-03481 | - | Z | + | + |
| Medium for lactic acid bacteria | - | - | - | - |

| | | | | |
|---|---|---|---|---|
| (Note)+: presence of antibacterial activity, -: absence of antibacterial activity | | | | |

All the bacterial cell preparations of NITE BP-03198, NITE BP-03199, NITE BP-03200, and NITE BP-03202, each having a plantaricin gene, and the bacterial cell preparations of ATCC-11454 and NITE ABP-03481, each having a nisin gene, were demonstrated to have antibacterial activity to *Lactococcus garvieae* of type I and type II.

Table 14 shows the types of plantaricin or nisin produced by NITE BP-03198, NITE BP-03199, NITE BP-03200, NITE BP-03201, NITE BP-03202, and NITE ABP-03481, and the amino acid sequences of them. As compared with standard plantaricin A, plantaricin A from NITE BP-03200 has substitution of one amino acid residue (underlined).

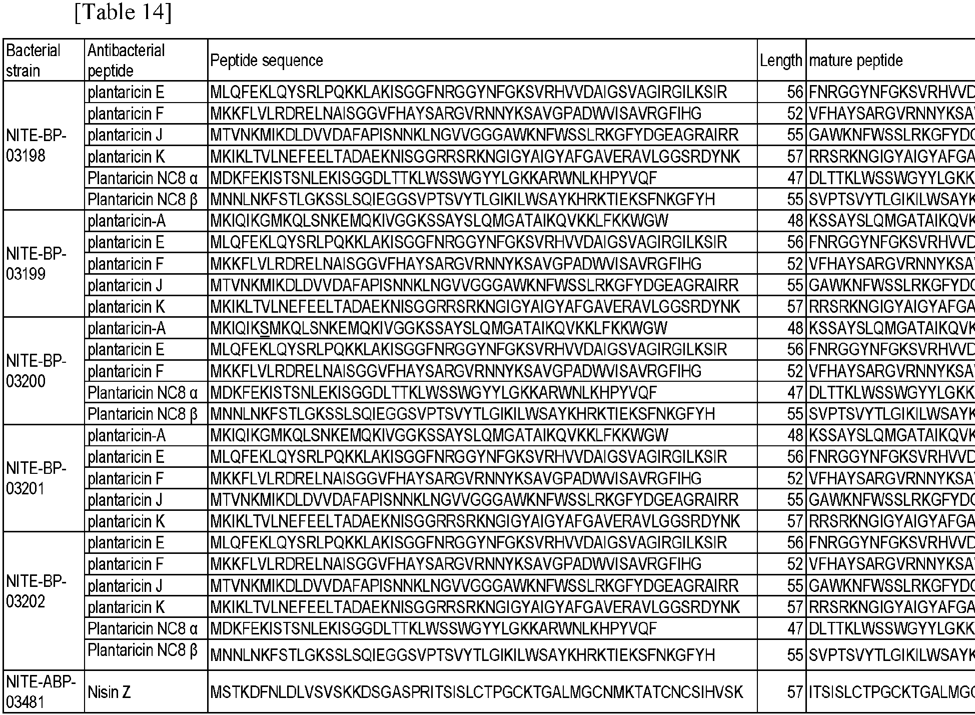

### [Experiment 8: Test for evaluating antibacterial activities of synthetic plantaricins]

Examination was performed on whether synthetic plantaricins inhibit the growth of *Lactococcus garvieae* type I (TUMSAT-K9408 strain) and type II (TUMSAT-LgN1 strain). The method in Experiment 8 is the same as that in Experiment 7 except that synthetic plantaricins were used in place of the lactic acid bacterial cell preparations. Each synthetic plantaricin was synthesized by using common techniques of organic synthesis. A solution containing plantaricin A (plantaricin A), a solution obtained by mixing plantaricin E and plantaricin F at a ratio (mole ratio) of 1:1 (plantaricin EF), a solution obtained by mixing plantaricin J and plantaricin K at a ratio (mole ratio) of 1:1 (plantaricin JK), or a solution obtained by mixing plantaricin NC8α and plantaricin NC8β at a ratio (mole ratio) of 1:1 (plantaricin NC8αβ) was added to a bacterial solution containing *Lactococcus garvieae,* and shaking culture was performed at 25°C for 8 hours. The bacterial solutions containing *Lactococcus garvieae* after culture were each dropped on agar medium, and whether a colony of *Lactococcus garvieae* formed was observed. The results are shown in Table 15.

**[Table 15]**

| | Plantaricin A | | | Plantaricin EF | | | Plantaricin JK | | | Plantaricin NC8αβ | | |
|---|---|---|---|---|---|---|---|---|---|---|---|---|
| | 1µM | 3µM | 10µM | 1µM | 3µM | 10µM | 1µM | 3µM | 10µM | 1µM | 3µM | 10µM |
| *Lactococcus garvieae* TUMSAT-K9408 | - | - | + | + | + | + | - | - | - | + | + | + |
| *Lactococcus garvieae* TUMSAT-LgN1 | - | + | + | + | + | + | - | + | + | + | + | + |

| | | | | | | | | | | | | |
|---|---|---|---|---|---|---|---|---|---|---|---|---|
| (Note)+: presence of antibacterial activity, -: absence of antibacterial activity | | | | | | | | | | | | |

Plantaricin A, plantaricin EF, plantaricin JK, and plantaricin NC8αβ all inhibited the growth of *Lactococcus garvieae* type II. Plantaricin A, plantaricin EF, and plantaricin NC8αβ inhibited the growth of *Lactococcus garvieae* type I. It was demonstrated that the synthesized plantaricins have antibacterial activity to *Lactococcus garvieae.*

### [Experiment 9: Test for evaluating antibacterial activity of purified product of nisin A]

Examination was performed on whether a purified product of nisin A inhibits the growth of *Lactococcus garvieae* type I (TUMSAT-K9408 strain) and type II (TUMSAT-LgN1 strain). The method in Experiment 9 is the same as those in Experiments 7 and 8 except that a purified product of nisin A was used in place of the lactic acid bacterial cell preparations or the synthetic plantaricins. A purified product of nisin A (manufactured by Sigma-Aldrich Co. LLC) was dissolved in water and removed of insoluble components by centrifugation, giving a nisin A solution. The nisin A solution was added to a bacterial solution containing *Lactococcus garvieae,* and shaking culture was performed at 25°C for 8 hours. The bacterial solution containing *Lactococcus garvieae* after culture was dropped on agar medium, and whether a colony of *Lactococcus garvieae* formed was observed. The results are shown in Table 16.

**[Table 16]**

| | Nisin A | | |
|---|---|---|---|
| | 1µM | 3µM | 10µM |
| *Lactococcus garvieae* TUMSAT-K9408 | + | + | + |
| *Lactococcus garvieae* TUMSAT-LgN1 | + | + | + |

| | | | |
|---|---|---|---|
| (Note)+: presence of antibacterial activity, -: absence of antibacterial activity | | | |

Nisin A inhibited the growth of *Lactococcus garvieae* type I and type II. It was demonstrated that the purified product of nisin A has antibacterial activity to *Lactococcus garvieae.*

### [Experiment 10: Examination of preventive or therapeutic effect of oral administration of lactic acid bacterium for streptococcosis in fish]

Examination was performed on whether oral administration of a lactic acid bacterial cell preparation reduces the death caused by streptococcosis in Japanese yellowtail. Juvenile fish (mojako) of Japanese yellowtail were distributed to four test sections, and the following feeds were given.
(1) Control: common feed (Kinko No. 3 from HIGASHIMARU CO., LTD.)
(2) Feed soaked in membrane-filtration concentrate of culture supernatant of NITE BP-03198 (10-fold concentrate, 0.3 mL/g)
(3) Feed soaked in salting-out concentrate of culture supernatant of NITE ABP-03481 (10-fold concentrate, 0.3 mL/g)
(4) Feed soaked in living cell suspension of NITE BP-03198 (1.2 × 10⁹ cfu/g)

The membrane-filtration concentrate of culture supernatant of NITE BP-03198 was prepared as follows. First, the lactic acid bacterium was cultured in MRS Broth at a temperature of 30°C, and then centrifuged to precipitate bacterial cells, giving a culture supernatant. The culture supernatant obtained was 10-fold concentrated with a microfiltration membrane (Synder Filtration, Inc., PES30), giving a membrane-filtration concentrate of the culture supernatant. The salting-out concentrate of culture supernatant of NITE ABP-03481 was prepared as follows. First, sodium chloride was added to the culture supernatant to reach a final concentration of 5.5 M, and the culture supernatant was stirred at 4°C overnight. Thereafter, the culture supernatant was centrifuged to give a precipitate. The precipitate obtained was dissolved in 10 µM aqueous solution of hydrochloric acid (pH 5), giving a salting-out concentrate of the culture supernatant. The salting-out concentrate corresponds to a solution 10-fold concentrated with reference to the culture supernatant before adding sodium chloride. The living cell suspension was prepared as follows. NITE BP-03198 was cultured in MRS liquid medium at a temperature of 30°C, and frozen glycerol stock (4 × 10⁹ cfu/mL, 3 mL/tube) was produced. The frozen glycerol stock was thawed and centrifuged to remove the supernatant, and the resultant was then resuspended in 3 mL of PBS to prepare a living cell suspension.

Juvenile fish of Japanese yellowtail were grown for habituation for approximately 1 month in an aquarium in which artificial seawater (Tetra, Marine Salt) was circulated, and then test was initiated. The temperature of the rearing water was 23 to 25°C. To each test section, 23 juvenile fish of Japanese yellowtail were provided. The estimated body weight of the juvenile fish of Japanese yellowtail at the initiation of the test was approximately 5 g. The feeds were each administered at 10 g/day/section in two separate portions per day. Two weeks after the initiation of providing, the Japanese yellowtail were soaked in rearing water containing 10¹⁰ cfu/mL of *Lactococcus garvieae* type II (LgN1 strain) for 3 hours to infect the fish with *Lactococcus garvieae.* After the infection, the Japanese yellowtail were returned to the former aquariums, and rearing was further continued for 2 weeks with administration of the feeds. The number of surviving Japanese yellowtail after the test is shown in Table 17.

**[Table 17]**

| Test section | Number of surviving individuals (fish) | Survival rate |
|---|---|---|
| (1)Control | 12 | 52% |
| (2)Culture supernatant of NITE BP-03198 | 17 | 74% |
| (3)Culture supernatant of NITE ABP-03481 | 22 | 96% |
| (4)Living cells of NITE BP-03198 | 17 | 74% |

For each test section, samples were collected from the brains and kidneys of 10 surviving fish 2 weeks after the infection by using a loop for isolation of bacteria, and seeded on agar medium to isolate bacteria. The agar medium used was a medium obtained by adding glucose to Mueller-Hinton medium to reach a final concentration of 0.2%. The grown bacteria were placed on a slide, and checked on whether *Lactococcus* type II was detected by using rabbit antiserum for *Lactococcus* type II (donated by Professor Yoshida, University of Miyazaki). The results are shown in Table 18.

**[Table 18]**

| Test section | Number of individuals with pathogenic bacterium detected (fish) |
|---|---|
| (1)Control | 3 |
| (2)Culture supernatant of NITE BP-03198 | 0 |
| (3)Culture supernatant of NITE ABP-03481 | 1 |
| (4)Living cells of NITE BP-03198 | 0 |

It was demonstrated that oral administration of a bacterial cell preparation of a bacterium having a plantaricin gene or a nisin gene can reduce the death caused by streptococcosis in an organism of fish. It was also demonstrated that infection with *Lactococcus garvieae* in organisms of fish was inhibited through oral administration of a bacterial cell preparation of a bacterium having a plantaricin gene or a nisin gene.

### REFERENCE SIGNS LIST

10 TH medium; 11 Lactic acid bacterial cell preparation; 12 Bacterial solution containing *Lactococcus garvieae;* 13 Bacterial culture solution; 14 TH agar medium; 15 Colony.

| | | |
|---|---|---|
| 4 | The indications below relate to the microorganism or other biological material described in the detailed description of the invention. | |
| 4-1 | Paragraph Number | 0025 |
| 4-3 | Indication of deposit | |
| 4-3-1 | Name of depositary institution | NPMD, National Institute of Technology and Evaluation, Patent Microorganisms Depositary (NPMD) |
| 4-3-2 | Address of depositary institution | Room 122, 2-5-8 Kazusakamatari, Kisarazu-shi, Chiba-ken, Japan 292-0818 |
| 4-3-3 | Date of deposit | April 9, 2020 (09.04.2020) |
| 4-3-4 | Accession Number | NPMD NITE BP-03201 |
| 4-5 | Designated states for which indications are made | All designated States |
| 5 | The indications below relate to the microorganism or other biological material described in the detailed description of the invention. | |
| 5-1 | Paragraph Number | 0025 |
| 5-3 | Indication of deposit | |
| 5-3-1 | Name of depositary institution | NPMD, National Institute of Technology and Evaluation, Patent Microorganisms Depositary (NPMD) |
| 5-3-2 | Address of depositary institution | Room 122, 2-5-8 Kazusakamatari, Kisarazu-shi, Chiba-ken, Japan 292-0818 |
| 5-3-3 | Date of deposit | April 9, 2020 (09.04.2020) |
| 5-3-4 | Accession Number | NPMD NITE BP-03202 |
| 5-5 | Designated states for which indications are made | All designated States |
| 6 | The indications below relate to the microorganism or other biological material described in the detailed description of the invention. | |
| 6-1 | Paragraph Number | 0026 |
| 6-3 | Indication of deposit | |
| 6-3-1 | Name of depositary institution | NPMD, National Institute of Technology and Evaluation, Patent Microorganisms Depositary (NPMD) |
| 6-3-2 | Address of depositary institution | Room 122, 2-5-8 Kazusakamatari, Kisarazu-shi, Chiba-ken, Japan 292-0818 |
| 6-3-3 | Date of deposit | June 9, 2021 (09.06.2021) |
| 6-3-4 | Accession Number | NPMD NITE BP-03481 |
| 6-5 | Designated states for which indications are made | All designated States |

| For receiving Office use only | | |
|---|---|---|
| 0-4 | This sheet was received with the international application (Yes/No) | Yes |
| 0-4-1 | Authorized officer | Tsuneo NAGAI |

## Claims

1. A composition for rearing an organism of fish, comprising at least one selected from plantaricin and nisin.

2. A composition for preventing or treating a disease caused by *Lactococcus garvieae* in fish, comprising at least one selected from plantaricin and nisin.

3. The composition according to claim 1 or 2, wherein
the plantaricin is at least one selected from plantaricin A, plantaricin E, plantaricin F, plantaricin J, plantaricin K, plantaricin NC8α, and plantaricin NC8β, and
the nisin is at least one selected from nisin A and nisin Z.

4. The composition according to claim 1 or 2, wherein the plantaricin contains any sequence of (a) to (c) and contains a peptide having antibacterial activity:
(a) an amino acid sequence set forth in any of SEQ ID NOs: 1 to 7,
(b) an amino acid sequence having 85% or more identity with an amino acid sequence set forth in any of SEQ ID NOs: 1 to 7, and
(c) an amino acid sequence formed by deletion, substitution, insertion, and/or addition of 1 or more and 15 or less amino acid residues in an amino acid sequence set forth in any of SEQ ID NOs: 1 to 7.

5. A composition for rearing an organism of fish, comprising bacterial cells or a cell culture product of a bacterium having at least one selected from a plantaricin gene and a nisin gene, or an extract thereof.

6. A composition for preventing or treating a disease caused by *Lactococcus garvieae* in fish, comprising bacterial cells or a cell culture product of a bacterium having at least one selected from a plantaricin gene and a nisin gene, or an extract thereof.

7. The composition according to claim 5 or 6, wherein the bacterium having at least one selected from the plantaricin gene and the nisin gene is at least one selected from NITE-BP-03198, NITE-BP-03199, NITE-BP-03200, NITE-BP-03201, NITE-BP-03202, and NITE-ABP-03481.

8. The composition according to claim 5 or 6, wherein
the plantaricin gene is at least one selected from a plantaricin A gene, a plantaricin E gene, a plantaricin F gene, a plantaricin J gene, a plantaricin K gene, a plantaricin NC8α gene, and a plantaricin NC8β gene, and
the nisin gene is at least one selected from a nisin A gene and a nisin Z gene.

9. The composition according to any one of claims 1, 2, 5, and 6, wherein the fish belongs to the order Perciformes or the order Salmoniformes.

10. The composition according to any one of claims 1, 2, 5, and 6, wherein the composition is a feed or a feed additive.

11. The composition according to any one of claims 1, 2, 5, and 6, wherein the composition is rearing water or a rearing water additive.

12. The composition according to any one of claims 1, 2, 5, and 6, wherein the composition is an injection.

13. A method for rearing an organism of fish, comprising giving the composition according to any one of claims 1, 2, 5, and 6 to an organism of fish.

14. A method for rearing an organism of fish, comprising administering at least one selected from plantaricin and nisin to an organism of fish.

15. A method for preventing or treating a disease caused by *Lactococcus garvieae* in fish, comprising administering at least one selected from plantaricin and nisin to an organism of fish.

16. The method according to claim 14 or 15, wherein the plantaricin is at least one selected from plantaricin A, plantaricin E, plantaricin F, plantaricin J, plantaricin K, plantaricin NC8α, and plantaricin NC8β, and
the nisin is at least one selected from nisin A and nisin Z.

17. The method according to claim 14 or 15, wherein the plantaricin contains any sequence of (a) to (c) and contains a peptide having antibacterial activity:
(a) an amino acid sequence set forth in any of SEQ ID NOs: 1 to 7,
(b) an amino acid sequence having 85% or more identity with an amino acid sequence set forth in any of SEQ ID NOs: 1 to 7, and
(c) an amino acid sequence formed by deletion, substitution, insertion, and/or addition of 1 or more and 15 or less amino acid residues in an amino acid sequence set forth in any of SEQ ID NOs: 1 to 7.

18. A method for rearing an organism of fish, comprising administering bacterial cells or a cell culture product of a bacterium having at least one selected from a plantaricin gene and a nisin gene, or an extract thereof to an organism of fish.

19. A method for preventing or treating a disease caused by *Lactococcus garvieae* in fish, comprising administering bacterial cells or a cell culture product of a bacterium having at least one selected from a plantaricin gene and a nisin gene, or an extract thereof to an organism of fish.

20. The method according to claim 18 or 19, wherein the bacterium having at least one selected from the plantaricin gene and the nisin gene is at least one selected from NITE-BP-03198, NITE-BP-03199, NITE-BP-03200, NITE-BP-03201, NITE-BP-03202, and NITE-ABP-03481.

21. The method according to claim 18 or 19, wherein
the plantaricin gene is at least one selected from a plantaricin A gene, a plantaricin E gene, a plantaricin F gene, a plantaricin J gene, a plantaricin K gene, a plantaricin NC8α gene, and a plantaricin NC8β gene, and
the nisin gene is at least one selected from a nisin A gene and a nisin Z gene.

22. The method according to any one of claims 14, 15, 18 and 19, wherein the administering is oral administration, soaking administration, or injection administration.

23. The method according to any one of claims 14, 15, 18 and 19, wherein the fish belongs to the order Perciformes or the order Salmoniformes.

24. A growth inhibitor or a bactericide for *Lactococcus garvieae,* comprising at least one selected from plantaricin and nisin.

25. A growth-inhibiting or bactericidal method for *Lactococcus garvieae,* comprising bringing at least one selected from plantaricin and nisin into contact with *Lactococcus garvieae.*

26. A growth inhibitor or a bactericide for *Lactococcus garvieae,* comprising bacterial cells or a cell culture product of a bacterium having at least one selected from a plantaricin gene and a nisin gene, or an extract thereof.

27. A growth-inhibiting or bactericidal method for *Lactococcus garvieae,* comprising bringing bacterial cells or a cell culture product of a bacterium having at least one selected from a plantaricin gene and a nisin gene, or an extract thereof into contact with *Lactococcus garvieae.*

28. A bacterium of Receipt Number: NITE-ABP-03481.

29. Bacterial cells or a bacterial cell culture of the bacterium according to claim 28, or an extract thereof.
